# DEMANDE DE BREVET EUROPEEN

(11) **EP 4 570 815 A1**
(43) Date de publication de la demande: **18.06.2025**
(21) Numéro de dépôt: 23307177.8
(22) Date de dépôt: 11.12.2023
(51) Int. Cl.: C07K 14/195, A23L 33/135, A61K 8/99, A61K 35/74, A61Q 19/00, A61Q 19/02, A61Q 19/08, C12N 1/20, C12N 15/09, C12N 15/74, C12P 1/04, C12Q 1/02, C12Q 1/04, C12Q 1/6883, C12Q 1/689

(54) **BACTERIE DU GENRE CORYNEBACTERIUM, ET SES UTILISATIONS COSMETIQUES**

(71) Demandeur: Laboratoires M & L, 04100 Manosque En Provence (FR); Université d'Aix Marseille, 13007 Marseille (FR); Assistance Publique - Hôpitaux de Marseille, 13005 Marseille (FR); Institut de Recherche pour le Développement, 13002 Marseille (FR)
(72) Inventeur: CENIZO, Valérie, 13650 Meyrargues (FR); MAUX, Mélinda, 04100 Manosque (FR); PORTES, Pascal, 13540 Puyricard (FR); LEMAIRE, Géraldine, 04220 Corbière en Provence (FR); COINUS, Tommy, 04800 Gréoux-les-Bains (FR); RIVOIRE, Stéphanie, 04100 Manosque (FR); BAZALINE, Niza, 13011 Marseille (FR); BOXBERGER, Manon, 13010 Marseille (FR); LA SCOLA, Bernard, 13109 Simiane-Collongue (FR); CASSIR, Nadim, 13016 Marseille (FR)
(74) Mandataire: Cabinet Becker et Associés

(57) **Abrégé**

La présente invention concerne une nouvelle bactérie du genre *Corynebacterium,* utile pour une application cosmétique. Une méthode de criblage de substances anti-âge est également proposée.

## Description

La présente invention concerne l'utilisation cosmétique d'une espèce bactérienne du genre *Corynebacterium* dont une souche a nouvellement été isolée.

### Etat de la technique

La peau est un écosystème habité par une pléthore de microorganismes aussi différents que des bactéries, des archées, des champignons ainsi que par des petits arthropodes. Ensemble, ils constituent le microbiote cutané. Ce microbiote est soumis à de nombreux paramètres de variation, à la fois intrinsèques et extrinsèques. Des études récentes ont identifié des corrélations entre les changements de la composition des populations commensales de la peau et l'état physiologique de cet environnement influencé par des facteurs tels que le vieillissement.

Des études de métagénomique ont mis à jour un grand nombre de bactéries, et ont notamment démontré la diminution, au cours du vieillissement, de *Cutibacterium acnés,* une des espèces les plus abondantes du microbiote cutané. Toutefois, ces études de métagénomique sont limitées à la caractérisation des bactéries abondantes, ce qui introduit des biais dans l'analyse de la composition du microbiote. Utilisée seule, la métagénomique ne peut donc suffire à refléter la complexité et la diversité du microbiote cutané.

La culturomique peut aider à pallier les inconvénients de la métagénomique. Cette technique repose sur la multiplication des conditions de culture de micro-organismes, permettant ainsi la croissance d'une plus grande diversité de bactéries, levures, ou champignons dont certains sont considérés comme non cultivables. Ces espèces ne sont pas nécessairement minoritaires dans cet habitat et leur influence est largement méconnue. L'une des principales difficultés de la culturomique cependant est la détermination des conditions d'isolement et de culture favorables à la croissance de certaines souches bactériennes jusqu'à présent méconnues. En effet, selon la zone cutanée considérée, il existe des différences de température, d'humidité ou de pH, qui peuvent influencer la composition bactérienne du microbiote.

### Résumé de l'invention

Les inventeurs sont parvenus à isoler une souche d'une nouvelle espèce bactérienne, dont la présence est corrélée à l'état de vieillissement de la peau. Cette souche a été déposée à la Collection Nationale de Cultures de Microorganismes (CNCM, Institut Pasteur, 25-28 rue du Docteur Roux, 75015 Paris, France) le 4 mai 2021 sous le numéro **I-5678.**

Un objet de l'invention est une bactérie, ladite bactérie appartenant à l'espèce *Corynebacterium cassirii* sp. nov. caractérisée en ce que son génome i) contient un gène *rpoB* présentant au moins 95,9% d'identité avec la séquence nucléotidique SEQ ID NO : 1 et/ou ii) présente une identité nucléotidique moyenne (ANI) d'au moins 95% avec la séquence génomique complète de ladite souche déposée à la CNCM sous le numéro **I-5678.**

De préférence, la bactérie est la souche isolée et déposée à la Collection Nationale de Cultures de Microorganismes (CNCM, Institut Pasteur, 25-28 rue du Docteur Roux, 75015 Paris, France) le 4 mai 2021 sous le numéro **I-5678.**

La bactérie utile selon l'invention peut être sous forme entière, viable, inactivée, ou morte.

Un autre objet de l'invention porte sur le lysat, ou fraction(s) de lysat de cette bactérie, ou son sécrétome.

L'invention vise également de manière générale l'utilisation cosmétique de ladite bactérie, pour le soin de la peau, ladite bactérie étant utilisée sous forme entière et viable, inactivée, ou morte, ou sous forme de lysat, ou fraction(s) de lysat, ou sous forme d'un ou plusieurs de ses métabolites, de préférence sous forme de son sécrétome.

Il est fourni ici une composition cosmétique à usage topique comprenant ladite bactérie isolée, sous forme entière, viable, inactivée, ou morte, ou ledit lysat ou fraction(s) de lysat, et comprenant éventuellement en outre au moins un actif anti-âge autre que ladite bactérie. Une composition cosmétique à usage topique est également fournie, comprenant un ou plusieurs métabolites de ladite bactérie, de préférence son sécrétome, et comprenant éventuellement en outre au moins un autre actif anti-âge.

De préférence lesdites compositions sont utiles pour tout soin de la peau, ou pour un effet cosmétique anti-âge ou un effet cosmétique hydratant. Ainsi elles sont particulièrement utiles pour lutter contre, c'est-à-dire prévenir et/ou traiter les signes de vieillissement cutané.

La composition utilisée selon l'invention peut être appliquée sur au moins une zone du corps d'une personne présentant des signes de vieillissement cutané (par exemple d'au moins 30 ans, voire d'au moins 40 ans), et plus particulièrement sur le visage, le cou et/ou le décolleté. En variante, elle peut être appliquée sur les mains ou le corps.

Un autre objet de l'invention est une méthode cosmétique de soin de la peau, comprenant l'application topique sur la peau d'une telle composition.

Encore un autre objet de l'invention est une méthode de criblage de substances susceptibles de présenter un effet cosmétique anti-âge, ladite méthode comprenant la mise en contact d'une peau humaine saine avec une substance candidate, et le suivi de la présence et/ou de la quantification de ladite bactérie de l'espèce *Corynebacterium cassirii* sp. nov., à la surface de la peau, par ce en quoi une substance qui stimule la prolifération de la bactérie à la surface de la peau est identifiée comme susceptible de présenter un effet cosmétique anti-âge.

L'invention fournit également une méthode pour évaluer l'état de vieillissement d'une peau humaine, ladite méthode comprenant l'identification et/ou la quantification de ladite bactérie de l'espèce *Corynebacterium cassirii* sp. nov., à la surface de la peau, la présence de ladite bactérie étant corrélée à une peau jeune et/ou sans rides et/ou sans taches.

Enfin l'invention a pour objet une utilisation de ladite bactérie de l'espèce *Corynebacterium cassirii* sp. nov. comme marqueur d'un vieillissement cutané, étant entendu que la présence de ladite bactérie est corrélée à une peau jeune et/ou sans rides et/ou sans taches.

### Légendes des figures

**Figure 1****. Evolution du portage de *C.cassirii* sp. nov. en fonction de l'âge des sujets.** Pourcentage de volontaires du groupe jeune et du groupe âgé chez lesquels *C.cassirii* sp.nov. a été détectée par PCR quantitative . **p=0.0067(test exact de Fisher)
**Figure 2****. Croissance de *C.cassirii* sp. nov. sur les peaux Labskin.** QPCR (barre gris clair), PMA QPCR (barre gris foncé) et dénombrement sur gélose (barre hachurée). Chaque barre représente la moyenne de 2 ou 3 échantillons (points) par condition.

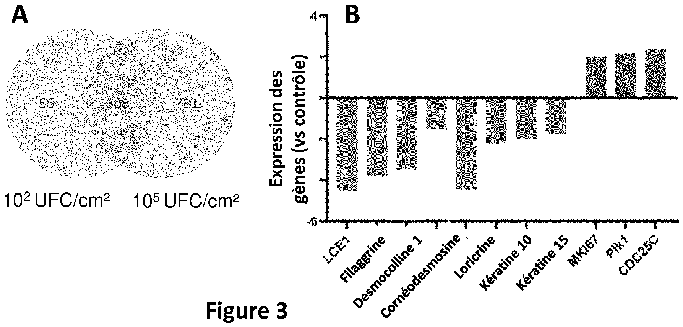

**Figure 3****. Analyse transcriptomique.** A- Diagramme de Venn représentant des gènes spécifiquement régulés par *C.cassirii* sp. nov. à 10² UFC/cm² ou 10⁵ UFC/cm² après 5 jours de coculture et en comparaison avec un contrôle sans coculture. Les gènes régulés par les deux conditions apparaissent à l'intersection des cercles. B- Histogramme des gènes régulés significativement en condition d'inoculation de 10⁵ UFC/cm² en comparaison avec le contrôle sans coculture à 5 jours de maintien en culture et impliqués dans la différenciation épidermique (gris clair) et la prolifération cellulaire (gris foncé). Un niveau d'expression ≥2 reflète une augmentation et ≤-2 une inhibition. Moderated t-Test p<0,05.
**Figure 4****. Analyse métabolomique.** Analyse bidimensionnelle en composantes principales entre les conditions contrôle et *C.cassirii* sp. nov. à J2 et à J5.

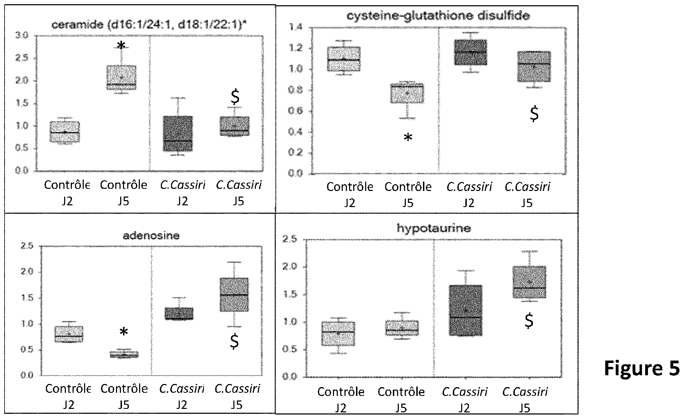

**Figure 5****. Exemples de métabolites significativement régulés par *C.cassirii* sp. nov. après 2 ou 5 jours de coculture sur le modèle de peau.** L'axe des ordonnées représente la quantité relative du métabolite entre les échantillons de chaque condition. Les données sont en effet normalisées de telle sorte que la valeur médiane de tous les échantillons de l'étude soit fixée à 1. Le graphique en boîtes à moustaches est utilisé pour représenter la répartition des données avec les 50 % des données médianes représentées par les boîtes (2^{ème} et 3^{ème} quartile) et les barres (ou moustaches) indiquant l'étendue complète des données. La ligne horizontale signale la valeur médiane (4 échantillons par condition) tandis que la moyenne est représentée par le signe +. Les comparaisons statistiques sont réalisées entre J2 et J5 pour la peau contrôle et entre la peau inoculée avec *C.cassirii* sp. nov. et la peau contrôle à J5. La significativité des variations est indiquée par le symbole * et $ respectivement.

### Description détaillée de l'invention

Les inventeurs sont parvenus à isoler une souche appartenant à une nouvelle espèce bactérienne, et en ont déterminé son caractère commensal. Par une étude clinique sur volontaires jeunes et âgés, les inventeurs ont mis en évidence la diminution significative de la présence de cette espèce au cours du vieillissement cutané. Elle s'est en outre avérée associée de manière significative avec des paramètres cliniques caractéristiques d'une peau jeune (moins de rides et de taches pigmentaires, un éclat et une homogénéité du teint supérieurs ainsi que des propriétés mécaniques telles que la fermeté et l'élasticité, supérieures).

Des investigations plus poussées sur son rôle et son interaction avec la peau, via la coculture utilisant des modèles cutanés 3D, ont permis de mettre en évidence son rôle bénéfique pour la peau et l'intérêt de stimuler sa croissance sur la peau, notamment âgée.

### Nouvelle espèce bactérienne et application cosmétique

Cette bactérie, du genre *Corynebacterium,* est l'espèce C. *cassirii* sp. nov. dont une souche nouvellement isolée a été déposée à la CNCM le 4 mai 2021 sous le numéro I-5678.

Le génome de la bactérie isolée et déposée à la CNCM le 4 mai 2021 sous le numéro I-5678 a été entièrement séquencé.

L'appartenance d'une bactérie à cette espèce est définie en ce que son génome i) contient un gène *rpoB* présentant au moins 95,9% d'identité avec la séquence nucléotidique SEQ ID NO : 1 et/ou ii) présente une identité nucléotidique moyenne (ANI) d'au moins 95% par rapport au génome complet de la souche déposée auprès de la CNCM le 4 mai 2021 sous le numéro I-5678.

Le terme "identité de séquence" ou "identité" désigne ici le nombre (%) de correspondances (nucléotiques identiques) dans les positions d'un alignement de deux séquences nucléotidiques. L'identité de séquence est déterminée en comparant les séquences lorsqu'elles sont alignées de manière à maximiser le chevauchement et l'identité tout en minimisant les écarts de séquence. En particulier, l'identité de séquence peut être déterminée à l'aide d'un certain nombre d'algorithmes mathématiques d'alignement global ou local, en fonction de la longueur des deux séquences. Les séquences de longueur similaire sont de préférence alignées à l'aide d'un algorithme d'alignement global (par exemple, l'algorithme de Needleman et Wunsch ; Needleman et Wunsch, J Mol Biol, 1970 ;48(3):443-53) qui aligne les séquences de manière optimale sur toute la longueur, tandis que les séquences de longueur sensiblement différente sont de préférence alignées à l'aide d'un algorithme d'alignement local (par exemple, l'algorithme de Smith et Waterman (Smith et Waterman, J Mol Biol, 1981 ; 147(1):195-7.) ou l'algorithme d'Altschul (Altschul et al, Nucleic Acids Res. 1997;25(17):3389-402; Altschul et al, FEBS J, 2005;272(20):5101-9). L'alignement aux fins de la détermination du pourcentage d'identité des séquences nucléotidiques peut être réalisé de diverses manières qui relèvent de l'art, par exemple en utilisant des logiciels accessibles au public disponibles sur des sites internet tels que https://blast.ncbi.nlm.nih.gov/Blast.cgi ou http://www.ebi.ac.uk/Tools/emboss/. L'homme du métier peut déterminer les paramètres appropriés pour mesurer l'alignement, y compris les algorithmes nécessaires pour obtenir un alignement maximal sur toute la longueur des séquences comparées.

Le séquençage du gène rpoB est une méthode simple pour discriminer les espèces du genre *Corynebacterium* (Khamis et al, J. Clin. Microbiology, 2005, 1934-1936).

La séquence du gène *rpoB* peut être amplifiée notamment grâce aux amorces dégénérées de séquences SEQ ID NO : 2 et SEQ ID NO : 3, décrites ci-dessous. Les amorces dites « dégénérées » sont une manière de décrire, à l'aide d'une séquence unique, un mélange de plusieurs amorces non dégénérées aux séquences différentes. La nomenclature internationale comporte les bases normales G (Guanine), A (Adénine), C (Cytosine) et T (Thymine) qui permettent d'écrire la séquence des amorces non dégénérées. Quand sur une position on peut avoir plusieurs bases différentes, on introduit des lettres qui précisent ce qu'est le mélange comme par exemple Y= T ou C, R = G ou A, W = A ou T, B = G ou T ou C. Par exemple, une séquence contenant la base dégénérée R résultera dans un produit dont environ 50 % des brins d'ADN auront un G dans cette position et les autres 50 % posséderont un A. Les amorces dites dégénérées, qui sont en réalité des mélanges de séquences d'amorces non dégénérées, permettent d'avoir dans le mélange toutes les amorces susceptibles de s'hybrider au gène rpoB de toutes les Corynébactéries.

| Séquence de l'amorce sens (5'→3') | Séquence de l'amorce antisens (5'→3') |
|---|---|
| CGWATGAACATYGGBCAGGT (SEQ ID NO : 2) | TCCATYTCRCCRAARCGCTG (SEQ ID NO :3) |

De manière préférée, la bactérie contient un gène *rpoB* présentant au moins 95,9%, de préférence au moins 96%, ou au moins 96,5%, au moins 97%, au moins 97,5%, au moins 98%, au moins 98,5%, au moins 99%, au moins 99,5%, voire au moins 99,8% d'identité avec la séquence nucléotidique SEQ ID NO : 1.

L'ANI est une méthode de référence de comparaison par paire de séquences bactériennes sur génome entier, fonctionnant à la fois sur génome complet ou partiellement complété, afin de déterminer le niveau de parenté génétique entre les souches bactériennes et définir les espèces bactériennes. En particulier, l'ANI entre deux génomes bactériens est calculée par comparaison par paires de l'ensemble des séquences similaires entre les deux génomes et peut être déterminée, par exemple, en utilisant l'un quelconque des logiciels disponibles publiquement de calcul d'ANI, incluant, sans s'y limiter, OrthoANI (Lee et al, Int J Syst Evol Microbiol. 2016, 66(2):1100-1103) et JSpeciesWS (Richter et al, Bioinformatics, 2016, 32(6):929-931). Typiquement, les génomes de souches bactériennes appartenant à la même espèce présentent une ANI supérieure ou égale à 95% tandis que les génomes de souches bactériennes appartenant au même genre présentent une ANI supérieure ou égale à 80% (Jain et al, Nat Commun. 2018; 9: 5114).

La séquence génomique de la souche *Corynebacterium cassirii* sp. nov. déposée à la CNCM le 4 mai 2021 sous le numéro I-5678 a été obtenue par séquençage de novo permettant la génération de 20 scaffolds, présentés dans le listing de séquences comme les séquences SEQ ID NO : 4 à 23. La taille de séquence obtenue par alignement des 20 scaffolds est compatible avec la taille des génomes du genre *Corynebacterium* et est donc représentative du génome entier.

Les séquences SEQ ID NO : 4 à 23 permettent ainsi la comparaison par paire de séquences bactériennes sur génome entier, pour le calcul de l'ANI.

De manière préférée, la bactérie présente une identité nucléotidique moyenne (ANI) d'au moins 95%, de préférence au moins 95,5%, de préférence au moins 96%, ou au moins 96,5%, au moins 97%, au moins 97,5%, au moins 98%, au moins 98,5%, au moins 99%, au moins 99,5%, voire au moins 99,8% par rapport au génome complet de la souche déposée auprès de la CNCM le 4 mai 2021 sous le numéro I-5678.

L'appartenance d'une bactérie à cette espèce peut être en outre confirmée par une hybridation ADN-ADN (DDH), notamment *in silico,* d'au moins 70% entre l'ADN génomique de la bactérie et l'ADN génomique de la souche *C.cassirii* sp. nov. déposée à la CNCM sous le numéro I-5678 (Goris et al, Int. J. of Systematic and Evolutionary Microbiology, 2007, 57, 81-91).

La bactérie utile selon l'invention est typiquement sous forme isolée. Le terme « isolé » englobe toute entité, ici une bactérie, en dehors de son habitat naturel, qui a été (1) séparée d'au moins certains des composants auxquels elle était associée lors de sa production initiale (que ce soit dans la nature ou dans un cadre expérimental) et/ou (2) produite, préparée, purifiée et/ou fabriquée par la main de l'homme. Les bactéries isolées peuvent être séparées d'au moins 10 %, 20 %, 30 %, 40 %, 50 %, 60 %, 70 %, 80 %, 90 % ou plus des autres composants auxquels elles étaient initialement associées.

Selon la présente invention, ladite bactérie peut être notamment fournie sous forme entière, notamment viable et/ou inactivée, notamment morte, et/ou sous forme de lysat, et/ou sous forme de l'une ou plusieurs de ses fractions et/ou sous forme d'un ou plusieurs de ses métabolites, préférentiellement de son sécrétome.

Pour chacune de ses formes, la bactérie selon l'invention peut être isolée ou associée à son milieu de fermentation et / ou de culture.

Préférentiellement, la bactérie selon l'invention est associée à son milieu de fermentation et/ou de culture.

Au sens de la présente invention, on entend par « isolée », le fait d'être non mélangée à un ou des composé(s) susceptible(s) de lui être associé(s) dans son milieu de fermentation et/ou de croissance.

Au sens de la présente invention, on entend par « forme entière » sa forme native, une forme dans laquelle l'enveloppe bactérienne est intacte, par opposition à une forme lysée. Lorsqu'elle est fournie ou utilisée sous forme entière, la bactérie *Corynebacterium cassirii* sp. nov. peut être viable, inactivée ou morte.

Au sens de la présente invention, on entend par « viable » une bactérie de *Corynebacterium cassirii* sp. nov. selon la présente invention capable de former des colonies en culture.

La culture de toute souche de *Corynebacterium cassirii* sp. nov. sous forme entière viable pourra être effectuée selon toute méthode classiquement connue de l'homme du métier.

Un exemple de milieu de culture est une gélose tryptone soja (TSA), comprenant de préférence également du Tween 80 1%, ou un bouillon tryptone soja (TSB) comprenant de préférence également du Tween 80 1%.

Un autre exemple de milieu est la gélose coeur-cervelle (milieu BHI) ou la gélose Mueller-Hinton (milieu MH), également de préférence complémentés de Tween 80 1%.

Au sens de l'invention, on entend par "inactivée" une bactérie de *Corynebacterium cassirii* sp. nov. selon la présente invention qui n'est plus capable, de préférence définitivement, de former des colonies en culture.

L'inactivation de la bactérie *Corynebacterium cassirii* sp. nov. pourra être effectuée selon toute méthode classiquement connue de l'homme du métier. Elle peut notamment être inactivée par irradiation, traitement thermique ou, sous certaines conditions, par lyophilisation, par traitement haute pression ou par extrusion.

Selon un mode de réalisation particulier, une inactivation thermique peut être réalisée en incubant la bactérie *Corynebacterium cassirii* sp. nov. pendant une période de temps donnée, avantageusement d'environ 10s à 90 min, préférentiellement d'environ 15 minutes à une heure, et à une température avantageusement d'environ 60°C à 150 °C. Préférentiellement, elle sera incubée pendant environ 30 minutes à environ 80°C.

Au sens de l'invention, on entend par "morte", une bactérie de *Corynebacterium cassirii* sp. nov. selon la présente invention qui n'est plus capable, définitivement, de former des colonies en culture.

Au sens de l'invention, on entend par "lysat", un matériau obtenu à l'issue de la destruction ou dissolution de cellules biologiques par un phénomène dit de lyse cellulaire provoquant ainsi la libération des constituants biologiques intracellulaires naturellement contenus dans les cellules bactériennes considérées et de fragments de composants de la paroi bactérienne et de la membrane cytoplasmique. Le lysat mis en oeuvre est donc formé en l'ensemble par les constituants biologiques intracellulaires et des constituants des parois et membranes cytoplasmiques de la bactérie *C.cassirii* sp. nov. selon l'invention.

Le lysat peut être obtenu par toute méthode classiquement connue de l'homme du métier. Elle pourra notamment être obtenue par un choc osmotique, un choc thermique, par ultrasons, par lyse enzymatique, par tyndallisation, ou encore sous contrainte mécanique de type centrifugation, ou par augmentation de la pression ou des combinaisons de ces différentes technologies. Des protocoles sont donnés en Exemple 4.

Préférentiellement, le lysat est obtenu par combinaison d'action mécanique et d'augmentation de la pression.

Dans un mode de réalisation préférentiel de l'invention, le lysat est associé au milieu de culture et/ou de fermentation de la bactérie.

Au sens de la présente invention, on entend par « fractions », un fragment de la bactérie *Corynebacterium cassirii* sp. nov. selon la présente invention dotée d'un effet cosmétique, en particulier pour lutter contre les signes de vieillissement cutané. Cette fraction correspond à une partie non totale des constituants biologiques intracellulaires et des constituants des parois et membranes cellulaires obtenus par lyse de la bactérie *Corynebacterium cassirii* sp. nov. selon l'invention.

Selon un mode particulier de l'invention, les fractions peuvent être le protoplasme de la bactérie.

Dans un mode de réalisation préférentiel de l'invention, les fractions sont associées au milieu de culture et/ou de fermentation de la bactérie.

Au sens de la présente invention, on entend par « métabolites » une ou plusieurs molécules organiques et/ou inorganiques issues du métabolisme de la bactérie *Corynebacterium cassirii* sp. nov. selon la présente invention et dotée également de propriétés cosmétiques anti-âge. Préférentiellement, il s'agit du sécrétome de la bactérie *Corynebacterium cassirii* sp. nov.selon l'invention.

Dans un mode de réalisation particulier de l'invention, les métabolites sont associés au milieu de culture et/ou de fermentation de la bactérie.

Au sens de la présente invention, on entend par « sécrétome », l'ensemble des molécules organiques sécrétées et/ou inorganiques sécrétées par la bactérie *Corynebacterium cassirii* sp. nov. selon la présente invention.

Le sécrétome peut être obtenu par toute méthode classiquement connue de l'homme du métier.

Dans un mode de réalisation préférentiel de l'invention, le sécrétome est associé au milieu de culture et/ou de fermentation de la bactérie.

Selon un mode préférentiel de réalisation de l'invention, la bactérie *Corynebacterium cassirii* sp. nov. selon l'invention est utilisée sous forme entière viable et/ou sous forme de lysat.

Lorsqu'elle est utilisée sous forme entière vivante selon l'invention, et qu'elle est utilisée seule, sous forme d'ingrédient actif, la bactérie selon l'invention peut être sous forme sèche, c'est-à-dire sous forme de poudre, avantageusement dans de la maltodextrine, préférentiellement en une teneur en bactérie de 10% à 80% (p/p) en poids de la bactérie, préférentiellement de 30% à 70% (p/p), encore avantageusement de 40 à 60% (p/p) en poids de la bactérie par rapport au poids total de poudre.

Alternativement, la forme entière vivante selon l'invention peut être utilisée diluée dans un solvant. Préférentiellement, ce solvant contient moins de 20% (v/v) en volume d'eau, encore préférentiellement moins de 5% (v/v) en volume d'eau, encore préférentiellement le solvant ne contient pas d'eau. Très préférentiellement, ce solvant est une huile cosmétiquement acceptable.

Lorsqu'elle est utilisée sous forme entière inactivée, notamment morte, et/ou sous forme de lysat, et / ou sous forme de l'une ou plusieurs de ses fractions, et qu'elle est utilisée seule sous forme d'ingrédient actif, la bactérie selon l'invention peut être sous forme sèche, c'est-à-dire sous forme de poudre, avantageusement dans de la maltodextrine, préférentiellement en une teneur en bactérie de 5% à 80% (p/p) en poids de la bactérie, préférentiellement de 10% à 70%(p/p) en poids de la bactérie, très préférentiellement d'environ 20% à 50% (p/p) en poids de la bactérie par rapport au poids total de poudre, encore avantageusement de 10% à 50% (p/p) en poids de la bactérie par rapport au poids total de poudre.

Alternativement, les formes entières inactivées, notamment mortes, et/ou les lysats, et/ou les fractions, peuvent être utilisés solubles et/ou dilués dans un solvant, notamment polaire, tel que l'eau, un alcool, un polyol, un glycol tel que le pentylène glycol et/ou l'hexylène glycol et/ou le caprylyl glycol, ou un de leurs mélanges, préférentiellement un mélange hydroglycolique ou hydroalcoolique, encore préférentiellement comprenant un glycol choisi parmi l'hexylène glycol, le caprylyl glycol et leurs mélanges.

Selon l'invention, la bactérie selon l'invention peut être utilisée seule, sous forme d'ingrédient actif et/ou dans une composition cosmétique destiné(e) à une application par voie topique. Dans un autre mode de réalisation, la bactérie selon l'invention, ou son lysat ou fraction(s) de lysat, peut être incorporée dans une composition cosmétique comprenant au moins un excipient cosmétiquement acceptable.

Une composition cosmétique à usage topique comprenant la bactérie isolée ou un lysat, ou fraction(s) de lysat est ainsi également fournie.

L'invention porte également sur l'utilisation à des fins cosmétiques de l'un ou plusieurs métabolites de ladite bactérie, de préférence son sécrétome.

Au sens de la présente invention, on entend par « utilisation et/ou composition cosmétique », une utilisation et/ou composition non pharmaceutique, c'est-à-dire qui n'est pas destinée à un usage thérapeutique et est appliquée sur une partie du corps dite saine, en particulier sur une zone de la peau dite saine.

Au sens de la présente invention on entend par « peau saine », une zone de peau sur laquelle est appliquée la bactérie selon l'invention et dite « non pathologique » par un dermatologue, c'est-à-dire ne présentant pas d'infection, de cicatrice, de maladie ou d'affection cutanée telle que candidose, impétigo, psoriasis, eczéma, acné ou dermatite, ou de plaies ou de blessures.

Au sens de la présente invention, on entend d'un point de vue cosmétique un effet anti-âge, par la capacité de lutter, prévenir et/ou traiter les signes de vieillissement cutané, notamment réduire les rides, et/ou la présence de tâches, ou encore maintenir et/ou augmenter la fermeté et/ou l'élasticité de la peau.

Le vieillissement peut être typiquement un vieillissement chrono-induit, que l'on retrouve par exemple chez les peaux dites matures c'est-à-dire les peaux de personnes de plus de 40 ans.

Selon l'invention, la bactérie selon l'invention peut être utilisée seule, sous forme d'ingrédient actif et/ou dans une composition cosmétique destiné(e) à une application par voie topique.

Lorsqu'elle est utilisée sous forme entière vivante selon l'invention, et qu'elle est utilisée seule, sous forme d'ingrédient actif, la bactérie selon l'invention peut être sous forme sèche, c'est-à-dire sous forme de poudre, avantageusement dans de la maltodextrine, préférentiellement en une teneur en bactérie de 10% à 80% (p/p) en poids de la bactérie, préférentiellement de 30% à 70% (p/p), encore avantageusement de 40 à 60% (p/p) en poids de la bactérie par rapport au poids total de poudre.

Alternativement, la forme entière vivante selon l'invention peut être utilisée en suspension dans un solvant. Préférentiellement, ce solvant contient moins de 20% (v/v) en volume d'eau, encore préférentiellement moins de 5% (v/v) en volume d'eau, encore préférentiellement le solvant ne contient pas d'eau. Très préférentiellement, ce solvant est une huile cosmétiquement acceptable.

Lorsqu'elle est utilisée sous forme entière inactivée, notamment morte, et/ou sous forme de lysat, et/ou sous forme de l'une ou plusieurs de ses fractions, et/ou sous forme d'un ou plusieurs de ses métabolites, notamment de son sécrétome, et qu'elle est utilisée seule sous forme d'ingrédient actif, la bactérie selon l'invention peut être sous forme sèche, c'est-à-dire sous forme de poudre, avantageusement dans de la maltodextrine, préférentiellement en une teneur en bactérie de 5% à 80% (p/p) en poids de la bactérie, préférentiellement de 10% à 70%(p/p) en poids de la bactérie, très préférentiellement d'environ 20% à 50% (p/p) en poids de la bactérie par rapport au poids total de poudre, encore avantageusement de 10% à 50% (p/p) en poids de la bactérie par rapport au poids total de poudre.

Alternativement, les formes entières inactivées, notamment mortes, et/ou les lysats, et/ou les fractions, et/ou les métabolites, notamment le sécrétome selon l'invention, peuvent être utilisés en suspension, solubles et/ou dilués dans un solvant, notamment polaire, tel que l'eau, un alcool, un polyol, un glycol tel que le pentylène glycol et/ou l'hexylène glycol et/ou le caprylyl glycol, ou un de leurs mélanges, préférentiellement un mélange hydroglycolique ou hydroalcoolique, encore préférentiellement comprenant un glycol choisi parmi l'hexylène glycol, le caprylyl glycol et leurs mélanges.

Dans un autre mode de réalisation, la bactérie selon l'invention peut être incorporée dans une composition cosmétique comprenant au moins un excipient cosmétiquement acceptable.

On entend, au sens de la présente invention, par excipient «cosmétiquement acceptable », un composé et/ou solvant topiquement acceptable, c'est-à-dire n'induisant pas de réponse allergique au contact de la peau, non toxique, non instable, chimiquement.

Dans un mode de réalisation préférentiel de l'invention, la bactérie selon l'invention est présente dans la composition cosmétique en une teneur comprise entre 1×10⁻⁴% et 10% (v / v) en volume, préférentiellement entre 1×10⁻⁴% et 5% (v/v) en volume, encore avantageusement entre 1×10⁻³% et 3% (v / v) en volume, encore préférentiellement entre 0,1% et 2% (v / v) en volume, par rapport au volume total de la composition, ladite composition comprenant en outre au moins un excipient cosmétiquement acceptable.

Avantageusement, la bactérie selon l'invention est présente dans la composition cosmétique, à une concentration de 1×10⁻⁴% à 10% en poids, préférentiellement entre 1×10⁻⁴% et 5% en poids, encore avantageusement entre 1×10⁻³% et 0,5% en poids de préférence encore de 0,001 à 0,1%, par rapport au poids total de la composition, ladite composition comprenant en outre au moins un excipient cosmétiquement acceptable. Dans le cas de bactéries vivantes, on peut utiliser par exemple 10⁴ à 10⁸ ufc/g, de préférence 10⁵ à 10⁷ ou 10⁸ ufc/g par rapport au poids total de la composition.

De manière particulièrement avantageuse, lorsque la bactérie selon l'invention est utilisée sous forme d'un ou plusieurs de ses métabolites, notamment de son sécrétome, elle est présente dans la composition cosmétique et / ou dermatologique, à une concentration de 0,01% à 10%, de préférence de 0,05 à 5%, de préférence encore de 0,05 à 0,5% en poids, encore préférentiellement à une concentration d'environ 0,1% en poids, par rapport au poids total de la composition, ladite composition comprenant en outre au moins un excipient cosmétiquement acceptable.

La composition cosmétique de l'invention peut être une formulation solide, liquide ou semi-solide comme des crèmes, des laits, des baumes, des mousses, des lotions, des sérums, des gels ou des gels crème. La bactérie (ou lysat, fractions de lysat, métabolites et sécrétome) peut être incorporée dans différents produits destinés au soin et/ou au maquillage de la peau, tels que des soins hydratants ou des fonds de teint, ou au nettoyage de la peau, tels que des lotions, gels ou laits démaquillants, des masques, ou encore dans des produits de soin et/ou de lavage des cheveux, tels que des shampooings et après-shampooings, entre autres.

Les excipients utilisés pour former une phase aqueuse et/ou phase grasse au sein de la composition cosmétique sont choisis par l'homme du métier de manière usuelle. Typiquement une phase aqueuse comprend de l'eau et/ou par exemple au moins un constituant choisi parmi les polyols et les gélifiants aqueux. Lorsqu'elle est présente, la phase grasse peut comprendre une ou plusieurs huiles volatiles et/ou non volatiles, en particulier des huiles végétales.

La composition peut en outre contenir différents constituants qui peuvent être dispersés dans la phase grasse et/ou dans la phase aqueuse, pour autant que ceux-ci soient compatibles avec une application topique sur la peau.

Elle peut ainsi renfermer au moins un émulsionnant huile-dans-eau ou eau-dans-huile, généralement non ionique, tel que des esters de polyoxyéthylène, des esters de sorbitane éventuellement polyéthoxylés, des esters d'acides gras et de glycérol éventuellement polyéthoxylés, des éthers d'alcools gras et de sucre tels que les alkyl glucosides, et leurs mélanges. Les émulsionnants peuvent représenter de 2 à 10% et de préférence de 4 à 6% du poids total de la composition.

La composition peut également comprendre une ou plusieurs charges pulvérulentes, qui se présentent avantageusement sous forme de microparticules poreuses ou creuses, de préférence poreuses.

La composition peut en outre comprendre des additifs choisis notamment parmi des agents photoprotecteurs organiques et/ou inorganiques, actifs dans la lumière bleue et/ou l'UVA et/ou l'UVB ; des polymères filmogènes à base de polysaccharides, capables de former un film protecteur anti-pollution, tels que les produits commercialisés par SOLABIA sous les dénominations commerciales Pollustop^{®} et Solashield^{®} ; des agents desquamants tels que des α- et β-hydroxyacides; des particules exfoliantes ; des parfums ; des agents anti-oxydants; des agents séquestrants; des ajusteurs de pH; des conservateurs; des pigments; des colorants ; et leurs mélanges.

De nombreux ingrédients cosmétiquement actifs sont connus par l'homme du métier pour améliorer la santé et/ou l'apparence physique de la peau. L'homme du métier sait formuler les compositions cosmétiques pour obtenir les meilleurs effets. D'autre part, ces composés peuvent avoir un effet de synergie lorsqu'ils sont combinés les uns aux autres.

La composition peut ainsi renfermer en outre au moins un actif cosmétique, en particulier un autre actif adapté à prévenir et/ou traiter les rides, le relâchement cutané et/ou la formation de taches pigmentaires, qui peut notamment être choisi parmi les agents anti-radicalaires, les agents stimulant la différenciation et/ou la prolifération des kératinocytes et/ou des fibroblastes ; les agents stimulant la synthèse de glycosaminoglycanes et/ou de collagène et/ou de fibrilles d'ancrage dermo-épidermique et/ou des fibres élastiques; les agents prévenant la dégradation du collagène et/ou des glycosaminoglycanes et/ou des fibrilles d'ancrage dermo-épidermique et/ou des fibres élastiques ; les agents anti-glycation ; les agents dépigmentants et/ou inhibant la mélanogénèse ; et leurs mélanges.

Des exemples de tels actifs anti-âge sont notamment : l'acide ascorbique, ses sels, ses éthers et ses esters, notamment le glucoside d'ascorbyle ; l'adénosine ; le ribose ; les extraits de miel ; les protéines et glycoprotéines, extraites notamment d'amande douce ; les protéines végétales hydrolysées, notamment issues du riz, des graines d'hibiscus ou du lupin ; les polypeptides et les pseudodipeptides, tels que le chlorhydrate de carcinine, le palmitoyl pentapeptide-4 (Pal-Lys-Thr-Thr-Lys-Ser) et le palmitoyl tripeptide-38 commercialisés notamment par SEDERMA sous les dénominations commerciale Matrixyl^{®} 3000 et Matrixyl^{®} Synthe'6, respectivement, le palmitoyl tripeptide-8 commercialisé par la société LUCAS MEYER sous la référence commerciale Nutrazen^{®}, le pentapeptide-18 commercialisé par la société LIPOTEC sous la dénomination commerciale Leuphasyl^{®} Solution, le sh-decapeptide-9 commercialisé par la société SANDREAM sous la dénomination commerciale Neoendorphin^{®} et le palmitoyl hexapeptide-52 commercialisé par la société INFINITEC sous la référence commerciale X50 Myocept^{®} Powder ; les silanes tels que le mannuronate de méthylsilanol ; les arabinoxylanes, extraits en particulier de farine de seigle et les galactoarabinanes, issus notamment du mélèze ; l'acide hyaluronique et ses sels ; les polyphénols, extraits en particulier de mimosa ; les alpha-hydroxyacides, dont ceux extraits de citron; les extraits (généralement aqueux) de plantes telles que le trèfle d'eau, la pensée sauvage, la prêle des champs, la Mafane (Acmella oleracea), le chardon aux ânes (Onopordum acanthium), le millefeuille (Achillea millefolium, contenu notamment dans le produit Neurobiox^{®} de la société BASF), l'embelia (Embelia concinna, telle que commercialisée par la société SEPPIC), le figuier de Barbarie (Opuntia ficus indica, commercialisé notamment par MIBELLE AG BIOCHEMISTRY sous la dénomination commerciale AquaCacteen^{®}), la sauge (Salvia officinalis, vendue notamment par PROVITAL GROUP), Vitex negundo (commercialisé notamment par les LABORATOIRES EXPANSCIENCE sous la référence commerciale Neurovity^{®}), la châtaigne, la papaye, l'arganier, l'avoine, le tournesol, la pâquerette, la pivoine ou l'aneth ; les extraits aqueux d'algues et notamment de coralline, de janie rouge, d'Ungaria pinnatifada, d'Alaria esculenta ou de Nannochlorosis oculata ; les huiles essentielles, notamment de myrte ou d'immortelle ; les gluconates de zinc et/ou de cuivre ; et leurs mélanges.

En variante ou en plus, la composition utilisée selon l'invention peut comprendre au moins un agent tenseur. Il peut s'agir d'un polymère tenseur, capable de tendre la peau par action mécanique et de réduire ainsi l'apparence des rides et des ridules, en particulier d'un polysaccharide, notamment d'un extrait d'algue ou de plancton marin ou d'une gomme végétale. Il peut s'agir également d'un agent tenseur agissant par voie biologique du type « botox-like », par exemple, un extrait d'Acmella oleracea commercialisé sous le nom de Gatuline^{®} Expression par la société GATTEFOSSE ; un extrait de graines d'hibiscus commercialisé sous le nom de Myoxinol^{®} LS9736 par la société BASF BCS ou encore un peptide de type Acétyl Hexapeptide-8 commercialisé sous le nom d'Argireline^{®} par la société Li potée.

De façon avantageuse, la bactérie *Corynebacterium cassirii* sp. nov. (ou lysat, fractions de lysat, métabolites et sécrétome), ou la composition de l'invention est destinée à être appliquée sur tout ou partie du corps et/ou du visage et/ou du cuir chevelu, préférentiellement les jambes, les cuisses, les bras, le ventre, le décolleté, le cou, les aisselles, encore préférentiellement tout ou partie du visage, et préférentiellement les joues, le front, le menton, les lèvres, le contour des yeux.

De façon avantageuse, la bactérie (ou lysat, fractions de lysat, métabolites et sécrétome), et/ou la composition la comprenant est destinée à être appliquée sur une zone de peau saine présentant des rides ou ridules, un relâchement et/ou une zone de peau saine affaissée et/ou une zone de peau saine manquant de tonicité.

Ainsi, avantageusement la composition cosmétique est destinée à une application par voie topique sur la peau saine.

De manière avantageuse, la bactérie de *Corynebacterium cassirii* sp. nov. selon l'invention, préférentiellement sous la forme d'une composition cosmétique selon l'invention, est utilisée en application topique régulière et préférentiellement au moins une fois par jour, avantageusement deux fois par jour, pendant au moins 10 jours, préférentiellement pendant 20 jours, et encore préférentiellement pendant au moins 28 jours.

La composition utilisée selon l'invention permet de lutter contre les signes du vieillissement cutané que sont notamment les rides, le relâchement cutané, la perte de souplesse et/ou d'élasticité de la peau, et l'amincissement de la peau et notamment de l'épiderme, l'apparition de taches pigmentaires ou d'un teint hétérogène.

La composition utilisée selon l'invention permet également d'améliorer l'hydratation cutanée, en particulier elle permet de lutter contre la sécheresse de la peau.

Par « rides », on entend les sillons dus à une altération des structures dermiques, notamment à une diminution du nombre et/ou du diamètre des fibres élastiques dans le derme papillaire et/ou à une réduction du tissu adipeux sous-cutané, dont l'apparence peut être réduite par un épaississement de la peau obtenu en particulier, selon l'invention, par augmentation de la prolifération des cellules épidermiques.

Au sens de la présente invention, on entend par « voie topique », ou « application topique », l'application locale directe et/ou la vaporisation de la bactérie de *Corynebacterium cassirii* sp. nov. (ou lysat, fractions de lysat, métabolites et sécrétome), ou de la composition selon l'invention sur la surface de la zone de peau saine.

Les compositions cosmétiques peuvent être appliquées chez la femme ou l'homme, de préférence une femme. Le sujet peut être avantageusement un adulte de plus de 30 ans, ou plus de 40 ans, plus de 50 ans, ou entre 55 et 70 ans.

### Méthodes de suivi et de criblage

Les résultats obtenus mettent en évidence le potentiel de la nouvelle espèce de l'invention en tant que marqueur d'une peau jeune. La présence de ladite bactérie à la surface d'une peau saine est corrélée à une peau jeune et/ou sans rides et/ou sans taches.

A ce titre, un objet de l'invention concerne une méthode de criblage de substances susceptibles de présenter un effet cosmétique anti-âge.

Il peut s'agir par exemple de petites molécules synthétiques, seules ou en combinaison, ou d'extraits naturels, notamment d'extraits naturels de plantes.

Cette méthode comprend l'application topique des substances candidates sur une zone de la peau, et la détermination ou suivi de la présence et/ou de la quantité de bactéries de cette nouvelle espèce.

La détermination de la présence et/ou quantité de bactéries à la surface de la peau peut être réalisée de manière avantageuse par amplification de l'ADN génomique bactérien, par exemple par PCR quantitative (qPCR).

Selon un mode de réalisation particulier, le gène de la ferrédoxine bactérienne est amplifié, par exemple en utilisant les amorces de PCR décrites dans l'exemple 1.

Une substance candidate qui stimule la prolifération de la bactérie à la surface de la peau est identifiée comme susceptible de présenter un effet cosmétique anti-âge.

L'état de vieillissement d'une peau humaine peut être également évalué, en déterminant la présence et/ou la quantité de bactéries de cette nouvelle espèce de l'invention, sur une zone de peau saine, la présence de ladite bactérie étant corrélée à une peau jeune et/ou sans rides et/ou sans taches. En d'autres termes, la quantité de bactéries trouvée à la surface de la peau diminue tandis que la peau présente un état de vieillissement de plus en plus marqué.

Les exemples et figures illustrent l'invention, sans limiter la portée de l'invention.

### Exemple 1 : Isolement d'une nouvelle espèce bactérienne (étude clinique)

### Matériel et méthodes

### 1- Inclusion des volontaires

Les échantillons de peau ont été collectés auprès de 47 femmes en bonne santé vivant autour de Marseille dans deux tranches d'âge différentes : de 18 à 30 ans (groupe jeune) et de plus de 55 ans (groupe âgé).

De plus pour cette étude, des conditions supplémentaires de recrutement ont été ajoutées : ne pas avoir suivi de traitement antibiotique ou antihistaminique pendant au moins 1 mois, ne pas souffrir d'une pathologie cutanée telle que dermatite atopique ou psoriasis (déterminée par un examen dermatologique), ne pas avoir pris une douche ou s'être lavé le front depuis la veille (au moins 12 heures) et ne pas avoir appliqué de produit cosmétique depuis le dernier lavage sur la zone échantillonnée. Pour le groupe jeune, les femmes devaient suivre un traitement contraceptif, à l'exclusion des pilules hautement dosées ayant une action connue sur l'acné. Les participantes du groupe âgé devaient être ménopausées, ne devaient suivre aucun traitement hormonal substitutif et devaient présenter des signes de photo vieillissement (taches sur le visage, le teint jaune, les rides marquées etc.).

Les résultats obtenus de 47 volontaires pour l'analyse des échantillons du front, sont répartis en 23 volontaires du groupe jeune et 24 volontaires du groupe âgé.

Pour l'analyse du portage des bactéries d'intérêt à l'aide de la technique de qPCR, un groupe de 62 femmes a été inclus (nommé Registre), constitué de 33 femmes du groupe âgé et 29 femmes du groupe jeune qui ont été prélevées sur le front.

### 2- Prélèvement des échantillons de microbiote cutané

Les zones de front échantillonnées étaient de 10 cm². Les prélèvements ont été effectués en utilisant des écouvillons stériles imbibés du milieu de transport « Culture Top^{®} Transport Media » (C-top Ae-Ana, Eurobio, France) et de manière Z-Stroked. 2 écouvillons ont été utilisés simultanément pour le front pour la culturomique. Des écouvillons agités à l'air ont été réalisés comme contrôle négatif. Les échantillons ont été immédiatement mis en culture pour la culturomique. Les volontaires du groupe Registre ont été prélevés en utilisant la même méthode mais avec un unique écouvillon Fecal Transwab^{®} (Sigma-Aldrich, Saint-Quentin Fallavier, France). Après le prélèvement, ces derniers sont immédiatement conservés à -80 °C avant l'extraction d'ADN.

### 3- Identification des micro-organismes par spectrométrie de masse MALDI-TOF

Après la mise en culture, les micro-organismes isolés ont été identifiés à l'aide de technique MALDI-TOF. Les bactéries ont été directement déposées sur la cible MSP96 (Bruker Daltonics, GmbH, Brême, Allemagne) avec un contrôle positif *(Escherichia coli*) un contrôle négatif (la matrice), cette méthode est appelée « dépôt direct ». L'acquisition des données a été réalisée sur le MALDI Biotyper (système Microflex LT ; Bruker Daltonics GmbH, Brême, Allemagne) à l'aide du logiciel Flex ControlTM et du logiciel d'identification MALDI BioTyper RTC (Bruker Daltonics GmbH, Brême, Allemagne). Lorsque l'identification n'est pas rendue possible par cette technique, trois repiquages successifs de la bactérie sont réalisés de manière à la purifier au maximum. Pour chaque nouvel essai d'identification, une extraction à l'acide formique est réalisée, 1µL de surnageant est déposé sur la plaque. Cette méthode permet de générer un spectre protéique sans impureté et maximise les chances de rapprocher le spectre généré avec les spectres contenus dans la base de données.

### 4- Identification des micro-organismes par séquençage génomique

Dans le cas où de nouveaux échecs d'identification par la technique de MALDI-TOF, le séquençage via les méthodes de Next Génération Sequencing est réalisé avec l'IlluminaMiseq (Illumina Inc., San Diego, CA, USA) et la méthode Oxford Nanopore (Oxford Nanopore Technologies, Inc, Oxford, UK).

Séquençage du génome de *Corynebacterium cassirii* sp. nov.

L'ADN génomique (ADNg) de *Corynebacterium cassirii* sp. nov. (souche de l'invention) a été extrait en deux étapes : un traitement mécanique a d'abord été réalisé avec des billes de verre lavées à l'acide (G4649 Sigma, St Louis, MO, USA) à l'aide de l'appareil FastPrep-24 ^{™} 5G Grinder (mpBio, Santa Ana, CA, USA) avec une vitesse de rotation maximale (6.5 m/s) pendant un temps de 90s. Ensuite, après 30 minutes d'incubation de lysozyme à 37 °C, l'ADN a été extrait sur le biorobot EZ1 (QIAGEN, Valencia, CA, USA) avec le kit EZ1 DNA Tissues. L'ADNg de *Corynebacterium cassirii* sp. nov. a été quantifié par un test Qubit haute sensibilité (Life technologies, Carlsbad, CA, USA) à 0,2 ng/µl. L'ADN génomique a ensuite été séquencé avec la technologie MiSeq (Illumina Inc, San Diego, CA, USA). Pour préparer la bibliothèque d'extrémité appariée (« pair ends »), une dilution a été effectuée pour avoir1 ng du génome comme matrice d'entrée pour préparer la bibliothèque d'extrémité appariée. L'étape de « tagmentation » a permis de fragmenter et de marquer l'ADN via l'ancrage au niveau des extrémités cohésives débordantes d'une séquence de nucléotides universelle. Ensuite, une amplification par PCR à cycle limité (12 cycles) introduit des codes-barres à double index. Après purification sur billes AMPure XP (Beckman Coulter Inc, Fullerton, CA, USA), les banques ont ensuite été normalisées sur billes spécifiques selon le protocole Nextera XT (Illumina Inc., San Diego, CA, USA). Les bibliothèques normalisées ont été regroupées en une seule bibliothèque pour le séquençage sur l'appareil MiSeq. La génération automatisée de cluster et le séquençage des extrémités appariées avec des lectures d'index doubles ont été effectués en une seule exécution de 39 heures à 2x250 pb.

De sorte à améliorer la séquence générée par la technique Illumina, l'approche Oxford Nanopore a été réalisée pour le séquençage de l'ADN génomique via la méthode 1D via la technologie Minlon en utilisant le kit SQK-LSK109. La bibliothèque a été construite à partir de 1 µg d'ADN génomique sans fragmentation, ni réparation d'extrémité. Les adaptateurs ont été ligaturés aux deux extrémités de l'ADN génomique. Après purification sur billes AMPure XP (Beckman Coulter Inc, Fullerton, CA, USA), la librairie a été quantifiée par un test Qubit haute sensibilité (Life technologies, Carlsbad, CA, USA). 1353 pores actifs ont été détectés pour le séquençage et le flux de travail WIMP a été choisi pour l'analyse bio-informatique en direct. 655K reads en tant que données brutes ont été générées.

### Analyse du génome

L'annotation a été obtenue avec le logiciel Prokka Galaxy Version 1.14.5+galaxy0 en utilisant les paramètres par défaut à l'aide de la plateforme Online Galaxy. Les données de séquence du génome ont été téléchargées sur le Type (Strain) Genome Server (TYGS), qui est une plate-forme de bio-informatique gratuite disponible en ligne (https://tygs.dsmz.de) pour effectuer une analyse taxonomique complète basée sur le génome. La détermination des génomes de souches type (« type strain » = souche officielle) les plus proches a été effectuée de deux manières complémentaires. Tout d'abord, le génome de *Corynebacterium cassirii* sp. nov. (souche de l'invention) a été comparé à tous les génomes de souches types disponibles dans la base de données TYGS via l'algorithme MASH, qui permet une approximation rapide de la parenté inter génomique, et le choix des dix souches types avec les plus petites distances. Deuxièmement, un ensemble supplémentaire de dix souches types étroitement apparentées a été déterminé via les séquences génétiques de l'ADNr 16S. Celui-ci a été extrait du génome soumis en utilisant RNAmmer et chaque séquence a ensuite été BLASTée contre la séquence du gène de l'ADNr 16S de chacune des 12983 souches de type actuellement disponibles dans la base de données TYGS. La superposition des résultats de ces deux méthodes a permis d'obtenir le classement des 50 meilleures souches de type correspondant (selon le bit score) et des distances précises ont été calculées en utilisant l'approche Genome BLAST Distance Phylogeny (GBDP) avec l'algorithme « couverture » et la formule de distance d5. Ces distances ont finalement été utilisées pour déterminer les 10 génomes de souches de type les plus proches pour chacun des génomes utilisateurs. Toutes les comparaisons par paires parmi l'ensemble de génomes ont été effectuées en utilisant le GBDP et des distances inter génomiques précises déduites avec l'algorithme « rognage » (Trimming) et la formule de distance d5. Les calculs ont été effectués avec 100 itérations. Les valeurs numériques DDH et les intervalles de confiance ont été calculés en utilisant les paramètres recommandés par le logiciel GGDC v2.1. En complément, le degré de similarité génomique de la souche de l'invention avec des espèces proches a été estimé à l'aide du logiciel OrthoANI en utilisant les paramètres par défaut. Les gènes de résistance aux antibiotiques et la présence de protéines liées à la pathogenèse ont été étudiés à l'aide des outils ABRicate intégrant les bases de données CARD et VFDB à l'aide de la plateforme Online Galaxy. L'analyse de la synthèse de métabolites secondaires a été effectuée par le logiciel AntiSmash puis confortée par l'analyse des séquences protéiques de la base de données Kyoto Encyclopedia of Genes and Genomes Pathway (KEGG) en utilisant BLASTp avec un seuil de valeur e de 1E⁻³.

### 5- Analyse du portage de l'espèce dans la population

L'ADN de 62 échantillons issus de l'écouvillonnage cutané sur le front (volontaires du Registre) a été extrait en utilisant le Kit QlAamp 96 DNA QIAcube HT suivant les recommandations du fournisseur (Qiagen). La qPCR a été effectuée avec le kit LightCycler^{®} 480 Probes Master (Roche Life Science, Germany) selon les recommandations du fournisseur. Le système qPCR a été désigné en utilisant le logiciel PrimerBlast+ et vérifié en utilisant l'outil BlastN contre la base de données nr.

Le tableau 1 ci-dessous décrit le système PCR, comprenant un couple d'amorces spécifiques et une sonde, conçu pour amplifier spécifiquement l'ADN génomique de *Corynebacterium cassirii* sp. nov. en ciblant le gène de la ferrédoxine.

**Tableau 1 : Système qPCR conçu pour amplifier spécifiquement l'ADN de Corynebacterium cassirii sp. nov.**

| **Bactérie cible** | **Gène cible** | **Amorce sens** | **Amorce antisens** | **Sonde** |
|---|---|---|---|---|
| ***Corynebacterium cassirii* sp. nov. souche de l'invention** | Ferrédoxine | GACGAACGGAT GGTCGAAGT (SEQ ID NO: 24) | CGAGGCGATCTTC TACGAGG (SEQ ID NO: 25) | TCCACTCGTCC GGGGTGTCG-TAMRA (SEQ ID NO: 26) |

### RESULTATS

### 1- Isolement et identification d'une nouvelle espèce du microbiote cutané

128 espèces différentes ont pu être isolées à partir des prélèvements issus du front, réparties dans 55 genres différents. Les genres majoritaires du fronts identifiés sont *Staphylococcus* (isolés sur 100% des volontaires), *Cutibacterium* (76,60%) et *Streptococcus* (48,94%) dont les principaux représentants *Staphylococcus epidermidis, Cutibacterium acnes, Streptococcus mitisloralis.*

Parmi elles, une nouvelle espèce a pu être isolée sur la peau d'une volontaire de 60 ans. Le séquençage de son génome a révélé qu'il s'agissait d'une nouvelle espèce du genre *Corynebacterium* jamais décrite et nommée *Corynebacterium cassirii* sp. nov..

La souche cultivable de *Corynebacterium cassirii* sp. nov. a été déposée à la CNCM (collection nationale de culture de microorganismes de l'institut Pasteur) sous le numéro CNCM-I-5678.

### 2- Mise en évidence de la caractéristique commensale de la nouvelle espèce

L'analyse de portage sur les échantillons du Registre, grâce aux amorces et sonde PCR spécifiques, a permis de détecter la présence de cette nouvelle espèce et de déterminer sa commensalité. Pour cela, un seuil de portage de 20% a été fixé, c'est-à-dire que l'espèce devait être portée par au moins 20% de la population étudiée.

**Table 2 : Portage de C. cassirii sp. nov. sur les volontaires du Registre**

| | **Volontaires jeunes** | **Volontaires âgés** | **Population totale du registre** |
|---|---|---|---|
| Nombre de volontaires étudiés | 29 | 33 | 62 |
| Pourcentage de volontaires du groupe porteurs de la bactérie | 45% | 27,3% | 35,5% |

22 échantillons du Registre ont montré une positivité sur les 62, elle est donc présente en quantité détectable chez 35% de la population globale et majoritairement dans le groupe jeune. Ainsi, dans la population globale, elle est portée par 45% des individus jeunes contre 27% de la population âgée. La présence de la bactérie semble diminuer avec l'âge, bien que la différence ne soit pas significative sur cet échantillon (Pearson's Chi-squared test, p value=0,1). Afin de valider cette hypothèse, une seconde étude clinique portant sur 108 volontaires est réalisée (exemple 2).

### Exemple 2 : Etude de corrélations entre la présence de Corynebacterium cassirii sp. nov. et les paramètres cliniques

### Objectif

L'objectif principal de l'étude était d'analyser chez la femme adulte la présence de bactéries de l'espèce *Corynebacterium cassirii* sp. nov. sur la peau du visage. L'objectif secondaire était d'étudier les variations de cette espèce bactérienne en fonction de l'âge et des caractéristiques cliniques de la peau.

### Matériel et méthodes

### Etude clinique

Des prélèvements du microbiote cutané ont été réalisés sur le front de 108 sujets volontaires répartis en 52 volontaires du groupe jeune (18-30 ans) et 56 volontaires du groupe âgé (55-70 ans). A partir de ces prélèvements, une détection des bactéries de l'espèce *Corynebacterium cassirii* sp. nov. a été réalisée, en utilisant la technique de PCR quantitative. Des évaluations cliniques de l'état de la peau et des mesures de paramètres cliniques tels que le taux de sébum, le taux d'hydratation, la perte insensible en eau et les mesures des propriétés biomécaniques de la peau ont été menées sur le visage de l'ensemble des sujets volontaires.

Des analyses de corrélations ont été réalisées entre les données cliniques, l'âge des sujets volontaires et la quantité de bactéries de l'espèce *Corynebacterium cassirii* sp. nov. trouvées à la surface de la peau du visage.

### Paramètres d'évaluation

### Prélèvement du microbiote cutané

Le microbiote cutané a été prélevé sur une surface de 25 cm² au niveau du front. Les prélèvements ont été effectués à l'aide d'un écouvillon (Transwab^{®}) pour chaque zone, en frottant délicatement l'écouvillon sur la peau du front.

### Prise de photos

Des photographies numériques à haute résolution du visage ont été réalisées à l'aide d'une table de repositionnement HeadScan et du logiciel CameraScan (Orion Concept), au laboratoire COSNAT Provence. Les acquisitions standardisées des sujets volontaires ont été réalisées par un/une technicien(ne) clinique, de face et de profil (45° à gauche et 45° à droite) et avec les filtres polarisés croisés et polarisés parallèles, lors de la visite d'évaluation. Les acquisitions ont ensuite servi de support visuel pour les scorages cliniques réalisés à la fin de l'étude.

### Mesures cliniques

Certaines mesures cliniques sont des mesures instrumentales non invasives nécessitant un contact cutané. Elles ont été effectuées sur une zone du front distincte de la zone prélevée pour le microbiote, ou sur la tempe pour les propriétés mécaniques :
- Hydratation par Cornéométrie (Corneometer^{®} CM825 - COURAGE & KHAZAKA)
- Taux de sébum par Sébumétrie (Sebumeter^{®} SM 815 - COURAGE & KHAZAKA)
- Perte insensible en eau par Tewamétrie (Tewametre^{®} TM300)
- Propriétés biomécaniques de la peau par Cutométrie (Cutometer^{®} MPA 580 - COURAGE & KHAZAKA)

### Scorages cliniques

Les scorages cliniques ont été réalisés par les investigateurs dermatologues sur la base des photographies standardisées, réalisées pendant la visite d'inclusion. Les échelles de scorage utilisées sont les suivantes :
- Rides de la patte d'oie selon l'Atlas BAZIN, Atlas du vieillissement cutanée, Volume 1 p. 40, éditions MED'COM, 2007,
- Ridules sous oculaires selon l'Atlas BAZIN, Atlas du vieillissement cutanée, Volume 1 p. 40, éditions MED'COM, 2007,
- Taches pigmentaires selon l'Atlas BAZIN, Skin Aging Atlas, Volume 2 p.93, MED'COM publishing 2010 pour le visage,
- Homogénéité du teint (échelle interne) selon l'échelle suivante en 5 points : 1- teint très hétérogène, 2- teint peu homogène, 3- teint moyennement homogène, 4- teint homogène, 5- teint très homogène
- Eclat du teint (échelle interne) selon l'échelle suivante en 5 points : 1- teint terne, éteint, 2-teint peu éclatant et lumineux, 3- teint moyennement éclatant et lumineux, 4- teint éclatant et lumineux, 5- teint très éclatant et lumineux

### Analyse de Corynebacterium cassirii sp. nov. par PCR quantitative

Tous les échantillons de microbiote cutané ont été congelés et stockés à -80 °C immédiatement après prélèvement. Après décongélation, 20 µl de Protéinase K à 20 mg/ml ont été ajoutés à chaque échantillon et chauffés 1h à 50 °C au bain-marie puis concentrés au SpeedVac avant extraction de l'ADN à l'aide du kit QIAamp^{®} PowerFecal^{®} Pro DNA (Qiagen). Une PCR quantitative a ensuite été réalisée, grâce aux systèmes PCR spécifiques du génome de *Corynebacterium cassirii* sp. nov. (Tableau 1) et du TaqMan^{®} Fast Advanced Master Mix (Applied Biosystems). La réaction de PCR est réalisée grâce à l'appareil RT-PCR QuantStudio^{™} 7 Pro (Applied Biosystems) sur 50 cycles d'amplification.

### Analyse statistique

Les données cliniques ont été formatées et l'absence d'erreur vérifiée à l'aide du logiciel R v 4.2.1. Toutes les analyses statistiques ont été réalisées dans le logiciel R. L'analyse de variations cliniques entre les groupes de sujets jeunes et âgés a été réalisée pour toutes les variables continues en utilisant le test Wilcoxon signed-rank test, et les p-values ont été ajustées par la méthode Benjamini-Hochberg. Les corrélations entre variables continues ont également été recherchées en utilisant le coefficient de corrélation Pearson et les résultats ajustés selon la même méthode. Les potentielles valeurs aberrantes ont été recherchées en utilisant le test du Z-score.

Un modèle de régression multilinéaire a ensuite été construit, basé sur les variables continues (données cliniques et valeurs de PCR quantitative) en utilisant la fonction 'lm' du logiciel R et les paramètres 'age ~ all continuous variables' afin d'observer quelles variables expliquent le plus les différences observées entre les groupes d'âge différent et prendre en compte tout élément confondant.

Nous avons ensuite comparé les résultats de qPCR avec les variables cliniques (test apparié) avec le test de Wilcoxon, en ajustant les p-values comme précédemment. Pour les variables continues, nous avons recherché les corrélations entre la qPCR et la variable grâce à la corrélation de Spearman sur la population globale.

### Résultats

### Caractérisation de la population étudiée

L'analyse des caractéristiques cliniques de la population étudiée révèle que les deux groupes de sujets (groupe jeune et groupe âgé) sont significativement différents pour toutes les variables étudiées, à l'exception des valeurs de tewamétrie et de cornéométrie (Tableau 3). Ainsi, les sujets des deux groupes ne présentent pas de différences en termes de barrière cutanée (téwamétrie) ou d'hydratation cutanée (cornéométrie). En revanche, les sujets âgés présentent de manière significative : une moindre production de sébum, plus de taches pigmentaires, un teint plus terne et plus hétérogène, plus de rides sous-oculaires et au niveau de la patte d'oie (Tableau 3). Les mesures au cutomètre révèlent également que la peau des sujets du groupe âgé est en moyenne significativement moins ferme (valeurs R6 en hausse, témoignant d'une plus grande viscosité de la peau, associée à une perte en collagène) et moins élastique (valeur R1 en hausse (moins bon retour élastique) et valeurs R2, R5 et R7 (paramètres d'élasticité) en baisse) (Tableau 3).

Ces données témoignent du vieillissement cutané.

**Tableau 3 : Analyse statistique des variations cliniques entre les groupes de sujets jeunes et âgés. Les variations sont exprimées en pourcentage pour les mesures instrumentales ou en point pour les scorages cliniques (les scores pouvant aller de 1 à 5) (test Wilcoxon signed-rank test avec ajustement des valeurs de p par la méthode Benjamini-Hochberg). Le seuil de significativité est fixé à 0,05 de la valeur de p. ****p<0,0001.**

| **Variable** | **Variation dans le groupe âgé par rapport au groupe jeune** | **Valeur de p ajustée (BH)** | **Analyse statistique** |
|---|---|---|---|
| Tewamétrie | -8,20% | 0.11475 | non significatif |
| Cornéométrie | 5,20% | 0.135 | non significatif |
| Sébumétrie | -17,20% | 3.65E-06 | **** |
| Taches pigmentaires | 3 | 6.90E-16 | **** |
| Eclat du teint | -1 | 6.08E-16 | **** |
| Homogénéité du teint | -1 | 5.87E-10 | **** |
| Sévérité des rides de la patte d'oie | 3,2 | 3.18E-18 | **** |
| Sévérité des rides sous oculaires | 2,6 | 1.56E-17 | **** |
| cutométrie R0 | -12,6% | 0.000372 | **** |
| cutométrie R1 | -41,7% | 3.17E-12 | **** |
| cutométrie R2 | -38,1% | 2.57E-15 | **** |
| cutométrie R3 | -12,6% | 0.000372 | **** |
| cutométrie R4 | -41,7% | 3.17E-12 | **** |
| cutométrie R5 | -44,0% | 1.45E-15 | **** |
| cutométrie R6 | -47,3% | 5.29E-13 | **** |
| cutométrie R7 | -51,6% | 2.08E-16 | **** |
| cutométrie R8 | -47,3% | 5.61E-13 | **** |

Corrélations statistiques entre la quantité de *Corynebacterium cassirii* sp. nov. et les paramètres cliniques

### Evolution de la quantité de Corynebacterium cassirii sp. nov. en fonction de l'âge

Les échantillons n'atteignant pas le seuil de détection (Ct) avant les 50 cycles de qPCR ont été considérés comme négatifs (bactérie indétectable).

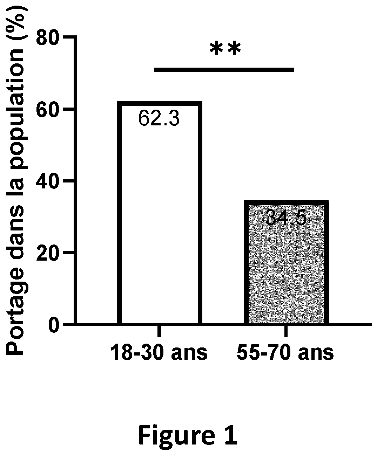

L'analyse du portage de *C.cassirii* sp. nov. dans les 108 échantillons montre que les volontaires du groupe âgé sont significativement moins porteurs de la bactérie (34.5% de la population âgée) que les volontaires du groupe jeune (62.3% de la population jeune)(Figure 1) Le portage est en effet 1.8 fois plus important chez les individus du groupe jeune (test exact de Fisher, p=0,0067) .

Par ailleurs, la quantité de *C.cassirii* sp. nov. détectée par qPCR a été corrélée aux paramètres cliniques. Ces résultats sont résumés dans le Tableau 4. Ils démontrent que *C.cassirii* sp. nov. diminue significativement avec l'augmentation de l'âge des volontaires, du score de tâches pigmentaires, des rides de la patte d'oie et des rides sous-oculaires. *C.cassirii* sp. nov. est en revanche associée à des scores d'éclat et d'homogénéité du teint plus élevés. Concernant les paramètres biomécaniques de la peau, *C.cassirii* sp. nov. est associée de manière significative avec les valeurs de R2, R5 et R7 plus élevées qui témoignent d'une peau plus élastique. *C.cassirii* sp. nov. n'est pas associée de manière significative à une variation des paramètres d'hydratation (cornéométrie), de barrière cutanée (téwamétrie) ou de taux de sébum.

Ces résultats confirment que *C.cassirii* sp. nov. est associée aux peaux jeunes, présentant une élasticité élevée, moins de rides, de tâches pigmentaires, ainsi qu'un teint plus homogène.

**Tableau 4 : Evolution de la quantité de C.cassirii sp. nov. en fonction des paramètres cliniques. Les coefficients de corrélation de Spearman sont indiqués ainsi que leur significativité pour chaque paramètre. ns=non significatif, *p<0,05, **p<0,01,***p<0,001, ****p<0,0001.**

| | **Variation de la quantité de *C.cassirii* sp. nov. en fonction du paramètre** | **Coefficient de corrélation R** | **Significativité (valeur de p) Spearman** |
|---|---|---|---|
| **Âge** | Diminution | -0,19 | ****p= 0,00038 |
| **Sébumètre** | Aucune | 0,14 | ns 0,15 |
| **Cornéomètre** | Aucune | -0,1 | ns p=0,3 |
| **Tewamètre** | Aucune | 0,11 | ns p=0,27 |
| **Taches pigmentaires** | Diminution | -0,19 | * p=0,048 |
| **Eclat du teint** | Augmentation | 0,29 | ** p=0,0028 |
| **Homogénéité du teint** | Aucune | 0,17 | ns p=0,072 |
| **Rides de la patte d'oie** | Diminution | -0,29 | ** p=0,0028 |
| **Rides sous-oculaires** | Diminution | -0,28 | ** p=0,003 |
| **R0** | Augmentation | 0,2 | * p=0,041 |
| **R1** | Aucune | -0,17 | ns p=0,081 |
| **R2** | Augmentation | 0,22 | * p=0,022 |
| **R3** | Augmentation | 0,2 | * p=0,041 |
| **R4** | Aucune | -0,17 | p=0,081 |
| **R5** | Augmentation | 0,23 | * p=0,016 |
| **R6** | Aucune | -0,15 | ns p=0,13 |
| **R7** | Augmentation | 0,23 | * p=0,014 |
| **R8** | Augmentation | 0,24 | * p=0,011 |

### Exemple 3 : Interactions entre Corynebacterium cassirii sp. nov. et la peau évaluées grâce à des modèles en co-culture

### Objectif

L'objectif de cette expérience était de comprendre les interactions de la souche de *Corynebacterium cassirii* sp. nov. avec la peau en utilisant un modèle co-culture *C.cassirii* sp. nov.-peau reconstruite. Les inventeurs ont suivi sa capacité à croître sur le modèle, témoignant de son adaptation à cet environnement. Puis l'analyse transcriptomique des peaux reconstruites visait à observer l'influence de la bactérie sur l'expression des gènes cutanés. Enfin, une analyse métabolomique de ces modèles a été menée afin de détecter les modifications métaboliques engendrées par la co-culture bactérie-peau.

### Matériel et méthodes

### Culture de Corynebacterium cassirii / sp. nov. :

La souche *C.cassirii* sp. nov. a été cultivée sur des géloses Tryptone Soja Agar (TSA) + Tween 80 (T80) 1% à 32°C pendant 3 jours. Quelques colonies ont été prélevées, suspendues en peptone-sel puis mises en préculture liquide dans un bouillon tryptone Soja (TSB) + T80 1% à 32°C sous agitation (180rpm) pendant 24 heures. Puis une culture liquide utilisant le même milieu de croissance a été réalisée en inoculant 5% de la préculture en milieu frais. Les bactéries en phase exponentielle de croissance ont été utilisées pour inoculer les modèles 3D de peau.

### Inoculation sur le modèle de peau reconstruite Labskin :

Le modèle de peau reconstruite Labskin (Innovenn, York, Angleterre) a été cultivé dès réception dans le milieu de culture du fournisseur pendant 24 heures à 37°C, 5% CO2. Le lendemain, 10² UFC/cm² ou 10⁵ UFC/cm² de *C.cassirii* sp. nov. ont été inoculées à la surface des peaux et la coculture a été poursuivie durant 2 ou 5 jours. Des modèles sans bactérie, sur lesquels seul le milieu de croissance de la bactérie a été ajouté en surface ont été utilisés comme contrôle.

### Évaluation de la croissance bactérienne

### Récupération de bactéries à partir de la surface du modèle de peau :

Les bactéries ont été récupérées de la surface des modèles de peau dans de l'eau physiologique contenant 0,1 % de T80 selon la méthode de Williamson & Kligman (Williamson P, Kligman AM. A new method for the quantitative investigation of cutaneous bacteria. J Invest Dermatol. 1965 Dec;45(6):498-503).

### Dénombrement sur gélose

La suspension bactérienne a été diluée en cascade d'un facteur 10 jusqu'à la dilution 10⁻⁸ et 100 µl de chaque dilution ont été inoculés sur des géloses TSA + T80 à 1 % et incubés pendant 3 jours à 32°C. Les colonies visibles dénombrées pour chaque dilution ont permis la quantification dans chaque échantillon.

### Q-PCR / PMA Q-PCR

Un volume de la suspension bactérienne récupérée de la surface des peaux a été remis en suspension dans la solution PowerBead (kit d'extraction, Qiagen, Courtaboeuf, France), et un autre a été prétraité avec 50 µM de PMAxx^{™} (Propidium monoazide PMAxx^{™}, Biotium, Fremont, CA, USA). L'échantillon a été placé dans l'obscurité à 4°C pendant 5 minutes, exposé à la lampe PhAST blue pendant 2 minutes et centrifugé à 10 000 g pendant 5 minutes. L'ADN a été extrait avec le kit DNeasy^{R} UltraClean^{R} Microbial (Qiagen). L'ADN de *C.cassirii* sp. nov. a été amplifié par PCR avec les amorces et sonde spécifiques définies dans la table 1 à 10 µM à l'aide du kit PowerTrack^{™} SYBR^{™} Green Master Mix (Applied Biosystems).

### Analyse transcriptomique

### Extraction d'ARN

L'épiderme des Labskin a été séparé du derme à l'aide d'une pince, broyé et homogénéisé à l'aide de l'homogénéisateur de l'Omni tissue homogenizer dans le réactif TRIzol^{®} (Invitrogen^{™}, Walthlam, Massachusetts, USA). La phase supérieure a été transférée sur des colonnes de centrifugation RNeasy et l'ARN total a été extrait à l'aide du kit Qiagen mini-RNeasy avec l'ajout d'une étape de digestion à la DNase.

### Préparation et hybridation des sondes

L'ARN total a été rétrotranscrit, amplifié et marqué avec de la cyanine 3 (Cy3) pour le kit de marquage One-Color Agilent Low Input Quick Amp (Agilent Technologies, Les Ulis, France). L'ARNc marqué a été hybridé sur les puces Agilent SurePrint G3 Human Gène Expression 8x60K v2 en utilisant les réactifs fournis dans le kit d'hybridation Agilent. Les lames ont été numérisées avec le scanner Agilent SureScan microarray scanning system. Les images à une couleur des puces ont été extraites avec le logiciel Feature Extraction (v12.0.0.7), pour la soustraction du bruit de fond et le contrôle qualité.

### Analyse de puces à ADN

Les données brutes des puces à ADN ont été normalisées et filtrées à l'aide du logiciel GeneSpring GX13.0. L'analyse des gènes régulés avec Ingenuity Pathway Analysis (IPA, Qiagen, Redwood City, CA) a permis l'identification des voies canoniques et leur classification en fonctions spécifiques. Les données ont été analysées par le test statistique Moderated t-Test avec un seuil de valeur de p<=0,05 et un seuil de régulation (fold-change) FC>=1,5.

### Analyse métabolomique

### Préparation des échantillons

Les échantillons d'épidermes de peaux colonisées ont été stockés à -80 °C jusqu'à leur traitement. Les échantillons ont été préparés à l'aide du système automatisé MicroLab STAR^{®} (Hamilton). Plusieurs standards ont été ajoutés avant la première étape du processus d'extraction à des fins de contrôle qualité. Les échantillons ont été extraits avec du méthanol sous agitation vigoureuse pendant 2 min (Glen Mills GenoGrinder 2000), suivie d'une centrifugation. L'extrait résultant a été divisé en quatre aliquots pour l'analyse par quatre méthodes différentes. Les échantillons ont été placés brièvement sur un TurboVap^{®} (Zymark) pour éliminer le solvant organique puis stockés pendant une nuit sous azote avant préparation pour analyse.

### Chromatographie liquide ultra-haute performance-spectroscopie de masse en tandem (UPLC-MS/MS) :

Toutes les méthodes ont utilisé une chromatographie liquide ultra-performance Waters ACQUITY (UPLC) et un spectromètre de masse haute résolution/précis Thermo Scientific Q-Exactive interfacé avec un appareil d'ionisation par électrospray chauffé (La source HESI-II) et l'analyseur de masse Orbitrap fonctionnaient à une résolution de masse de 35 000. L'extrait d'échantillon a été séché puis reconstitué dans des solvants compatibles avec chacune des quatre méthodes. Chaque solvant de reconstitution contenait une série d'étalons à des concentrations fixes pour assurer l'injection et la cohérence chromatographique.

Un aliquot a été analysé en utilisant des conditions d'ions positifs acides, méthode optimisée pour les composés plus hydrophiles. Dans cette méthode, l'extrait est élué d'une colonne C18 (Waters UPLC BEH C18-2.1x100 mm, 1,7 µm) en utilisant un gradient eau-méthanol, contenant 0,05 % d'acide perfluoropentanoïque (PFPA) et 0,1 % d'acide formique (FA). Un deuxième aliquot a été analysé en utilisant des conditions d'ions positifs acides mais la chromatographie a été optimisée pour les composés plus hydrophobes. Dans cette méthode, l'extrait a été élué de la colonne C18 en utilisant un gradient méthanol-acétonitrile-eau, 0,05 % de PFPA et 0,01 % de FA. Un troisième aliquot a été analysé dans des conditions de base optimisées pour les ions négatifs à l'aide d'une colonne C18 dédiée. Les extraits basiques ont été élués en utilisant un gradient méthanol-eau, avec du bicarbonate d'ammonium 6,5 mM à pH 8. Le quatrième aliquot a été analysé par ionisation négative après élution à partir d'une colonne HILIC (Waters UPLC BEH Amide 2,1x150 mm, 1,7 µm) en utilisant un gradient eau-acétonitrile avec 10 mM de formiate d'ammonium, pH 10,8. Pour l'analyse en spectrométrie de masse (MS), la plage de balayage a couvert environ 70-1000 m/z.

### Bioinformatique

Un logiciel propriétaire a été utilisé pour faire correspondre les ions à une bibliothèque interne de standards analytiques pour l'identification des métabolites et pour la quantification des métabolites par le calcul de l'aire sous le pic. Les fondations matérielles et logicielles de ces composants informatiques sont LAN backbone et les serveurs de base de données exécutant Oracle 10.2.0.1 Enterprise Edition.

### Analyse statistique

L'analyse statistique bidirectionnelle two-way ANOVA et l'analyse en composantes principales (ACP) ont été utilisées pour analyser les données.

### RESULTATS

### Croissance de C.cassirii sp. nov. sur un modèle de peau reconstruite

Dans cette étude, nous avons suivi la croissance bactérienne de *C.cassirii* sp. nov. sur les peaux. Pour cela, nous avons effectué un comptage bactérien au jour 0 (J0), au jour 2 (J2) et 5 (J5) post-inoculation.

Pour ce faire, nous avons utilisé les techniques de QPCR, PMA-QPCR et de dénombrement sur gélose pour maximiser la récupération des données. Ces différentes méthodes ont permis de dénombrer les bactéries totales, vivantes et mortes, grâce à la QPCR. La PMA-QPCR a quant à elle été utilisée pour ne dénombrer que les bactéries viables, dont la paroi était intacte au moment du prélèvement. Le PMAxx ne pénètre en effet qu'à l'intérieur des bactéries dont les parois cellulaires sont endommagées et se lie à leur ADN, empêchant ainsi l'amplification de l'ADN des bactéries mortes lors de la PCR. Enfin, le dénombrement des colonies sur gélose a permis de déterminer la quantité de bactéries viables et cultivables.

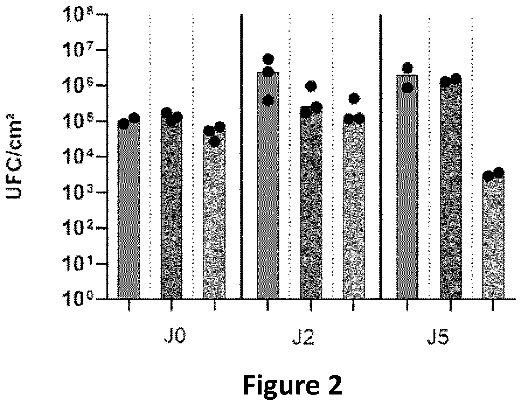

Au jour 0, qui correspond à l'inoculation sur les peaux, les 3 techniques ont permis de dénombrer le même nombre de bactéries, environ 10⁵ UFC/cm² (Figure 2). Puis après 2 jours de culture, une croissance bactérienne a pu être observée, avec une quantité de bactéries totales (2,6 10⁶ UFC/cm² par QPCR) légèrement supérieure à celle des bactéries viables (4,3 10⁵ UFC/cm² par PMA-QPCR) et cultivables (2,1 10⁵ UFC/cm² sur gélose). Cette différence est devenue plus prononcée au jour 5 avec les deux techniques de QPCR et PMA-QPCR dénombrant plus de 10⁶ UFC/cm² contre 2.10³ UFC/cm² de bactéries capables de former des colonies sur gélose. Ces résultats indiquent que *C.cassirii* sp. nov. s'est donc adaptée à l'environnement offert par la surface cutanée des modèles Labskin et a proliféré au cours de cette co-culture, essentiellement durant les 2 premiers jours. La décroissance observée à J5 suggère également que le nombre maximum de bactéries viables sur la surface du modèle (1cm²) est régulée autour de 10³ bactéries, alors que la PMA-QPCR en complément indique une quantité plus importante de bactéries viables mais non cultivables.

La croissance et la survie de *C.cassirii* sp. nov. sur le modèle de peau reconstruite nous a ensuite permis d'étudier la réponse du tissu cutané après 5 jours de coculture avec *C.cassirii* sp. nov. par analyse transcriptomique pour tenter de comprendre les interactions entre cette bactérie et la peau.

L'analyse transcriptomique à l'aide des logiciels Ingenuity Pathway (IPA) et Genespring a révélé que les bactéries à 10⁵ UFC/cm² ont spécifiquement régulé l'expression de 1089 contre 364 gènes pour la condition inoculée à 10² UFC/cm². Alors que 308 gènes ont été régulés par les deux concentrations bactériennes, 781 ont été spécifiquement régulés par la forte concentration tandis que 56 sont spécifiques de la concentration faible (Figure 3A). Par exemple, sur les 29 gènes impliqués dans la formation de la couche cornée et régulés par la concentration élevée, seuls 5 le sont également par la concentration faible. La condition d'inoculation de 10⁵ UFC/cm² a donc été choisie pour une analyse transcriptomique plus poussée.

Les voies de régulation impliquées dans la différenciation épidermique, la biosynthèse des lipides, la formation et le maintien de la couche cornée ont été inhibées en présence de bactéries par rapport au modèle contrôle (Figure 3B). En effet, l'expression des gènes codant pour des protéines du stratum corneum comme la late cornified envelope 1 (LCE1), la filaggrine, la desmocolline 1 ou la cornéodesmosine, a été diminuée en présence des bactéries, tout comme celle des gènes codant pour les kératines 1 et 10 retrouvées dans les couches supra basales de l'épiderme ainsi que la loricrine exprimée dans la couche granuleuse, une des dernières étapes de différenciation des kératinocytes. D'autre part, plusieurs gènes impliqués dans la prolifération cellulaire ont vu leur expression stimulée par la présence des bactéries. Par exemple, l'expression des gènes Ki-67, un marqueur de prolifération, ou Polo-Like Kinase 1 (Plk1) et cell division cycle 25C (CD25C), responsables de la division et de la prolifération cellulaire, a été stimulée.

Ces résultats indiquent que la présence de *C.cassirii* sp. nov. à la surface des peaux reconstruites favorise le renouvellement épidermique en induisant la prolifération de la couche basale.

### C.cassirii sp. nov. régule les voies métaboliques

Nous avons ensuite étudié l'interaction entre la peau et les bactéries par analyse métabolomique afin de visualiser les métabolites produits par la peau seule ou à la fois par les bactéries inoculées à 10⁵ UFC/cm² et la peau reconstruite dans le modèle de coculture. En effet, les métabolites sont le témoin de l'action des enzymes et protéines porteuses de la fonction codée par les gènes.

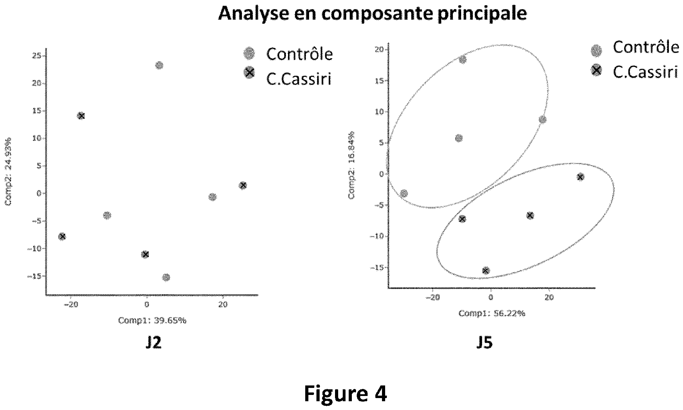

L'analyse en composantes principales a révélé une faible différence entre la peau témoin et la peau avec des bactéries au jour 2. Au jour 5 en revanche, une séparation nette a été observée entre les conditions contrôle et *C.cassirii* sp. nov. indiquant une composition différente des métabolites. (Figure 4).

L'analyse statistique a révélé un impact significatif du temps de culture sur les peaux contrôles avec 179 métabolites significativement différents (p<0.05) ainsi qu'en tendance pour 66 autres (p<0.1) (Tableau 5). Ceux-ci sont principalement liés à la différenciation épidermique du modèle qui se poursuit durant les 5 jours de culture. Ainsi, les métabolites appartenant aux voies liées au métabolisme des acides aminés, des peptides, des sucres, des nucléotides, des cofacteurs et des vitamines et notamment à la voie des lipides ont été régulées, ces derniers étant augmentés.

La présence de *C.cassirii* sp. nov. a spécifiquement régulé 123 métabolites (72 significatifs et 51 en tendance) (Tableau 5).

**Tableau 5 : Comparaison statistique du nombre de métabolites significativement régulés entre J2 et J5 pour le modèle contrôle, et à J5 entre la condition C.cassirii sp. nov. et le contrôle.**

| **Analyse ANOVA** | **Evolution entre J2 et J5 Peau contrôle** | ***C.cassiri* vs Contrôle à J5** |
|---|---|---|
| Nombre de métabolites augmentés ou diminués p<0.05 | 28 augmentés | 61 augmentés |
| | 151 diminués | 11 diminués |
| Nombre de métabolites augmentés ou diminués 0.05<p<0.10 | 7 augmentés | 49 augmentés |
| | 59 diminués | 2 diminués |

Tout d'abord, les quantités de lysophopholipides, de céramides, de cholestérol, d'acides gras libres, de monoacylglycérols et de diacylglycérols sont significativement moins élevés dans les modèles de peau avec *C.cassirii* sp. nov. par rapport au modèle contrôle à J5, car leur production a augmenté plus fortement avec le temps dans le modèle contrôle. L'exemple du céramide d16 :1/24 :1, d18 :1/22:1 est illustré dans la figure 5. Cela témoigne d'un ralentissement de la différenciation épidermique par rapport aux peaux contrôles.

Par ailleurs, la figure 5 illustre également une quantité significativement plus importante de cysteine-glutathion disulfide en présence de la bactérie, lequel baisse plus rapidement dans les modèles contrôles au cours du temps et qui suggère une augmentation du stress oxydant en présence de la bactérie. Il est important de noter, cependant, que la prolifération des cellules en elle-même génère des espèces réactives de l'oxygène (ROS) via la production d'énergie médiée par la phosphorylation oxydative. De plus, l'adénosine s'est avérée être la molécule la plus fortement augmentée dans les peaux inoculées avec *C.cassirii sp. nov.* (Figure 5). Il a été démontré que celle-ci favorise la prolifération des kératinocytes épidermiques humains normaux et par cette voie, favorise la cicatrisation. Ainsi, l'augmentation de l'oxydation observée dans les peaux inoculées par *C.cassirii sp. nov.* pourrait être expliquée par un niveau plus important de prolifération par rapport aux peaux contrôles, comme le confirme d'ailleurs l'analyse transcriptomique.

Enfin, un certain nombre d'osmolytes sont produits en quantité significativement plus importante au cours du temps dans les peaux contrôles mais parmi ceux-ci, l'hypotaurine est spécifiquement augmentée dans les peaux inoculées par *C.cassirii sp. nov.* Outre le maintien du volume et de l'hydratation des cellules, plusieurs autres fonctions ont été décrites pour les osmolytes, notamment l'amélioration de l'intégrité des jonctions serrées, la stabilisation des protéines et la protection contre les dommages cellulaires induits par les UV. Cette production d'osmolytes soutient l'utilisation de la bactérie pour modifier l'équilibre osmotique, conduisant à une meilleure rétention d'eau et une meilleure hydratation de la peau.

L'ensemble des analyses transcriptomique et métabolomique menées sur les modèles de coculture *peau-C.cassirii sp. nov.* indique donc qu'en présence de *C.cassirii sp. nov.,* la peau ralentit sa différenciation épidermique au profit de la prolifération. Le renouvellement épidermique, qui ralentit au cours du vieillissement cutané est donc stimulé, contribuant à diminuer les rides.

### Exemple 4 : Préparation de différentes formes de C.cassirii sp. nov.

### Production de bactéries vivantes

Préparation des précultures de *C.cassirii* sp. nov.

Les précultures sont préparées dans 10 mL de milieu MH, supplémentés en Tween 80 à 0.2% à partir d'une cryobille de *C.cassirii* sp. nov. conservée à -80°C. La préculture est incubée pendant 72h à 32°C, et sous agitation à 180 rpm.

Préparation des cultures de *C.cassirii* sp. nov.

Une culture de *C.cassirii* sp. nov. est préparée en tube Falcon en inoculant 10 à 20 mL de bouillon MH avec 400 µL de préculture. La culture est incubée à 32°C pendant 24h sous agitation à 180 rpm.

### Production de bactéries inactivées ou lysées

Les étapes suivantes sont réalisées à partir d'une culture de *C.cassirii* sp. nov. préparée comme il est décrit dans le paragraphe ci-dessus « production de bactéries vivantes ».

### Protocole de lavage des bactéries

La culture est centrifugée 30 min à 4 °C sous agitation à 4000 rpm. Le culot est repris dans 20 mL d'eau milliQ stérile. Un second lavage est réalisé (centrifugation de 30 min à 4 °C 4000 rpm). Le culot est repris dans 6 mL milliQ stérile. La suspension bactérienne est stockée dans la glace jusqu'à utilisation.

### Traitement

La suspension bactérienne est aliquotée en vue de l'application d'un traitement (lyse et/ou inactivation).

Différents traitements peuvent être appliqués : traitement physique (par action de la température, par ultra son, par action mécanique, par pression, ...) et traitement chimique (tampon de lyse, éthanol, hydroxyde de sodium, ...).

Les échantillons traités sont conservés à 4 °C jusqu'à vérification de l'inactivation des bactéries.

*Traitement thermique :* un traitement court de pasteurisation a été mis en place pour l'inactivation des bactéries : Un échantillon de suspension bactérienne est placé dans un bain marie à 60°C pendant 20 min ou 70 °C pendant 10 min. L'échantillon est refroidi dans la glace pendant 5 min.

Un autre traitement, plus long et à plus forte température a été testé pour favoriser la lyse des bactéries : Un échantillon de suspension bactérienne est placé dans un bain marie à 95 °C pendant 6h. L'échantillon est refroidi dans la glace pendant 5 min.

Des cycles de congélation et décongélation ont également été testés : Un échantillon de suspension bactérienne est soumis à 5 cycles de congélation à -70 °C et décongélation à température ambiante.

*Traitement chimique :* Un échantillon de suspension bactérienne est mis en présence de CTAB (bromure de cétyltriméthylammonium), tampon de lyse, pendant 30 min à 60°C. Ensuite du lysozyme à raison de 1mg/mL d'échantillon est ajouté. L'échantillon est incubé 15 min à 37°C. D'autres traitements chimiques testés sont le traitement à l'éthanol à 70% et le traitement à l'hydroxyde de sodium (6mg/mL).

D'autres traitements propres à l'inactivation de la bactérie tels que le traitement au formol ou le traitement au Béta-propiolactone sont également possibles.

*Traitement par ultra son :* Un échantillon de suspension bactérienne est déposé dans la cuve à ultra son, et soumis à un traitement aux ultra-sons pendant 80 min.

*Traitement par UVC* également possible.

*Traitement par broyeur à bille :* Un échantillon de suspension bactérienne est déposé dans un tube de 2mL contenant des billes en verre de 0,1mm de diamètre. L'étape de broyage consiste en 21 cycles de 30sec de broyage mécanique à 5000rpm.

*Traitement par homogénéiseur à haute pression* : Un échantillon de suspension bactérienne est déposé dans l'appareil pour être soumis à une pression comprise entre 1500 et 2000 Bar, préférentiellement 2000 Bar.

*Traitement par choc osmotique* : Un échantillon de suspension bactérienne est transféré dans un milieu hypotonique. Pour rétablir l'équilibre osmotique, l'eau pénètre dans les bactéries qui éclatent par rupture de leur membrane plasmique.

### Vérification de l'inactivation ou de la lyse des bactéries

*Examen microscopique* : les échantillons traités et non traités sont observés au microscope en contraste de phase (GTx1000) et après coloration de Gram (GTx1000) pour visualiser l'aspect de la suspension (bactéries intègres ou bactéries morcelées).

Les méthodes de microscopie électronique à balayage peuvent également être utilisées pour visualiser l'impact des traitements sur les bactéries.

*Examen macroscopique* : un ensemencement d'une boite de gélose COS est réalisé avec les échantillons traités et non traités et incubé à 32°C jusqu'à 96h. L'absence de culture permet de rendre compte de l'inactivation des bactéries.

*Examen moléculaire* : la mesure de la concentration en ADN extracellulaire au NanoDrop est réalisée à partir d'un échantillon traité et d'un échantillon non traité : l'échantillon traité est centrifugé à 16000g pendant 10 min pour l'analyse au NanoDrop. La différence de concentration en ADN extracellulaire entre les deux échantillons permet de rendre compte de l'intégrité des bactéries ou de leur lyse totale ou partielle après traitement.

D'autres méthodes existent telles que des marquages avec des intercalants de l'ADN (DAPI ou PMA).

### Exemple 5 : Exemples de composition cosmétique type crème visage comprenant la bactérie Corynebacterium cassirii sp. nov. selon l'invention

| | |
|---|---|
| Émulsionnants H/E | 6,00% |
| Corps gras | 15,00% |
| Gélifiants hydrophiles | 3,00% |
| Polyols | 3,50% |
| Actifs additionnels anti-âge | 5,50% |
| Actifs anti-séborrhéique | 3,00% |
| Hydroxyde de sodium | 1,00% |
| Huile essentielle | 0,03% |
| Extrait végétal | 0,50% |
| Parfums | Qs |
| Conservateurs | Qs |
| Anti-oxydants | Qs |
| Eau | Qsp |
| Bactérie encapsulée | 10⁻³% |

### Exemple 6 : Exemples de composition cosmétique type huile visage comprenant la bactérie Corynebacterium cassirii sp. nov. selon l'invention

| | |
|---|---|
| Ester d'acides gras | 54,00% |
| Alcool gras | 15,00% |
| Huile végétale | 20,00% |
| Alcane | 10,00% |
| Huile essentielle | 0,5% |
| Anti-oxydant | Qs |
| Parfums | Qs |
| Bactérie lyophilisée | 10⁻³% |

## Revendications

1. Bactérie appartenant à l'espèce *Corynebacterium cassirii sp. nov.* **caractérisée en ce que** son génome i) contient un gène rpoB présentant au moins 95,9% d'identité avec la séquence nucléotidique SEQ ID NO : 1 et/ou ii) présente une identité nucléotidique moyenne (ANI) d'au moins 95% par rapport au génome complet de la souche déposée auprès de la CNCM le 4 mai 2021 sous le numéro I-5678.

2. Bactérie selon la revendication 1, **caractérisée en ce qu'**il s'agit de la souche déposée auprès de la CNCM le 4 mai 2021 sous le numéro I-5678.

3. Bactérie selon la revendication 1 ou 2, qui est sous forme entière et viable, inactivée, ou morte.

4. Lysat, ou fraction(s) de lysat, ou sécrétome, de la bactérie telle que définie à la revendication 1 ou 2.

5. Composition cosmétique à usage topique comprenant la bactérie selon la revendication 1 ou 2, ou un lysat, ou fraction(s) de lysat, ou sécrétome, selon la revendication 4, ladite composition comprenant en outre optionnellement au moins un actif anti-âge autre que ladite bactérie.

6. Composition selon la revendication 5, ladite bactérie étant présente dans la composition à une concentration de 1×10⁻⁴% à 10% en poids, préférentiellement entre 1×10⁻⁴% et 5% en poids, encore avantageusement entre 1×10⁻³% et 0,5% en poids par rapport au poids total de la composition, ladite composition comprenant en outre au moins un excipient cosmétiquement acceptable.

7. Composition selon l'une des revendications 5 et 6, pour un effet cosmétique anti-âge ou un effet cosmétique hydratant.

8. Utilisation cosmétique d'une bactérie telle que définie à l'une des revendications 1 ou 2, pour le soin de la peau, ladite bactérie étant utilisée sous forme entière et viable, inactivée, ou morte, ou sous forme de lysat, ou fraction(s) de lysat, ou sous forme d'un ou plusieurs de ses métabolites, de préférence sous forme de son sécrétome,

9. Utilisation cosmétique selon la revendication 8, pour prévenir et/ou traiter les signes du vieillissement cutané.

10. Méthode cosmétique de soin de la peau, comprenant l'application topique sur la peau d'une composition selon l'une des revendications 5 ou 6.

11. Méthode cosmétique selon la revendication 10, dans laquelle l'application topique est effectuée sur tout ou partie du corps, du visage et/ou du cuir chevelu, préférentiellement les jambes, les cuisses, les bras, le ventre, le décolleté, le cou, les aisselles, encore préférentiellement tout ou partie du visage, et préférentiellement les joues, le front, le menton, les lèvres, le contour des yeux.

12. Méthode de criblage de substances susceptibles de présenter un effet cosmétique anti-âge, ladite méthode comprenant la mise en contact d'une peau humaine saine avec une substance candidate, et le suivi de la présence et/ou de la quantification d'une bactérie de l'espèce *C.cassirii sp. nov.* à la surface de la peau, par ce en quoi une substance qui stimule la prolifération de la bactérie à la surface de la peau est identifiée comme susceptible de présenter un effet cosmétique anti-âge ; l'espèce *C.cassirii sp. nov.* étant **caractérisée en ce que** son génome i) contient un gène rpoB présentant au moins 95,9% d'identité avec la séquence nucléotidique SEQ ID NO : 1 et/ou ii) présente une identité nucléotidique moyenne (ANI) d'au moins 95% par rapport au génome complet de la souche déposée auprès de la CNCM le 4 mai 2021 sous le numéro I-5678 ; ladite bactérie étant de préférence la souche déposée à la CNCM le 4 mai 2021 sous le numéro I-5678.

13. Méthode pour évaluer l'état de vieillissement d'une peau humaine, ladite méthode comprenant l'identification et/ou la quantification d'une bactérie de l'espèce *C.cassirii sp. nov.,* à la surface de la peau, la présence de ladite bactérie étant corrélée à une peau jeune et/ou sans rides et/ou sans taches ; l'espèce *C.cassirii sp. nov.* étant **caractérisée en ce que** son génome i) contient un gène rpoB présentant au moins 95,9% d'identité avec la séquence nucléotidique SEQ ID NO : 1 et/ou ii) présente une identité nucléotidique moyenne (ANI) d'au moins 95% par rapport au génome complet de la souche déposée auprès de la CNCM le 4 mai 2021 sous le numéro I-5678 ; ladite bactérie étant de préférence la souche déposée à la CNCM le 4 mai 2021 sous le numéro I-5678.

14. Méthode selon la revendication 12 ou 13, comprenant une quantification de ladite bactérie par PCR quantitative.

15. Utilisation d'une bactérie de l'espèce *C.cassirii sp. nov.,* comme marqueur d'un vieillissement cutané, étant entendu que la présence de ladite bactérie est corrélée à une peau jeune et/ou sans rides et/ou sans taches ; l'espèce C. *cassirii sp. nov.* étant **caractérisée en ce que** son génome i) contient un gène rpoB présentant au moins 95,9% d'identité avec la séquence nucléotidique SEQ ID NO : 1 et/ou ii) présente une identité nucléotidique moyenne (ANI) d'au moins 95% par rapport au génome complet de la souche déposée auprès de la CNCM le 4 mai 2021 sous le numéro I-5678 ; ladite bactérie étant de préférence la souche déposée à la CNCM le 4 mai 2021 sous le numéro I-5678.
